# EUROPEAN PATENT SPECIFICATION

(11) **EP 3 328 423 B1**
(45) Date of publication and mention of the grant of the patent: **26.05.2021**
(21) Application number: 16747758.7
(22) Date of filing: 27.07.2016
(51) Int. Cl.: A61K 39/12, A61K 39/39

(54) **ENHANCED IMMUNE RESPONSE IN FISH**
ERHÖHTE IMMUNREAKTION BEI FISCHE
AMÉLIORATION DE LA RÉPONSE IMMUNITAIRE DE POISSONS

(30) Priority: 31.07.2015 US 201562199840 P
(43) Date of publication of application: 06.06.2018
(73) Proprietor: Bayer Animal Health GmbH, 51373 Leverkusen (DE)
(72) Inventor: WEISS, Christian, 51381 Leverkusen (DE); SIMARD, Nathalie C., Penicuik Scotland EH26 0PZ (GB); KOESLING, Jan, 10439 Berlin (DE)
(74) Representative: Be IP
(86) International application number: PCT/EP2016/067850
(87) International publication number: WO 2017/021242

(56) References cited:
- EP-A2- 0 773 295
- WO-A1-99/56755
- WO-A1-2010/130374
- WO-A1-2012/032497
- WO-A1-2012/084951
- WO-A1-2015/128461
- WO-A2-99/66879
- RAMASAMY HARIKRISHNAN ET AL: "Poly,-lactide-co-glycolic acid-liposome encapsulated ODN on innate immunity inagainst", VETERINARY IMMUNOLOGY AND IMMUNOPATHOLOGY, ELSEVIER BV, AMSTERDAM, NL, vol. 147, no. 1, 4 April 2012 (2012-04-04) , pages 77-85, XP028423517, ISSN: 0165-2427, DOI: 10.1016/J.VETIMM.2012.04.008 [retrieved on 2012-04-11]
- JIE JI ET AL: "Nanodelivery Systems as New Tools for Immunostimulant or Vaccine Administration: Targeting the Fish Immune System", BIOLOGY, vol. 4, no. 4, 19 October 2015 (2015-10-19), pages 664-696, XP055309129, DOI: 10.3390/biology4040664
- GAËLLE VANDERMEULEN ET AL: "New Generation of Plasmid Backbones Devoid of Antibiotic Resistance Marker for Gene Therapy Trials", MOLECULAR THERAPY, NATURE PUBLISHING GROUP, GB , vol. 19, no. 11 1 November 2011 (2011-11-01), pages 1942-1949, XP002698168, ISSN: 1525-0016, DOI: 10.1038/MT.2011.182 Retrieved from the Internet: URL:http://www.nature.com/mt/journal/v19/n 11/full/mt2011182a.html [retrieved on 2011-08-30]
- ZAKS KAREN ET AL: "Efficient immunization and cross-priming by vaccine adjuvants containing TLR3 or TLR9 agonists complexed to cationic liposomes", THE JOURNAL OF IMMUNOLOGY, THE AMERICAN ASSOCIATION OF IMMUNOLOGISTS, US, vol. 176, no. 12, 15 June 2006 (2006-06-15), pages 7335-7345, XP002485614, ISSN: 0022-1767

### SEQUENCE LISTING

<110>
<120> ENHANCED IMMUNE RESPONSE IN AQUATIC SPECIES
<130> BAYR
<160> 17
<170> PatentIn version 3.5
<210> 1
   <211> 4242
   <212> DNA
   <213> Artificial sequence
<220>
   <223> Nucleotide sequence of plasmid pGCMB75.6
<400> 1
   tgaccgccca acgacccccg cccattgacg tcaataatga cgtatgttcc catagtaacg      60
   ccaataggga ctttccattg acgtcaatgg gtggagtatt tacggtaaac tgcccacttg     120
   gcagtacatc aagtgtatca tatgccaagt ccgcccccta ttgacgtcaa tgacggtaaa     180
   tggcccgcct ggcattatgc ccagtacatg accttacggg actttcctac ttggcagtac     240
   atctacgtat tagtcatcgc tattaccatg gtgatgcggt tttggcagta catcaatggg     300
   cgtggatagc ggtttgactc acggggattt ccaagtctcc accccattga cgtcaatggg     360
   agtttgtttt ggcaccaaaa tcaacgggac tttccaaaat gtcgtaacaa ctccgcccca     420
   ttgacgcaaa tgggcggtag gcgtgtacgg tgggaggtct atataagcag agctcgttta     480
   gtgaaccgtc agatcgcctg gagacgccat ccacgctgtt ttgacctcca tagaagacac     540
   cgggaccgat ccagcctccc ctcgaagccg atctgataac ggtaccgata agctggcggc     600
   cgattaagct acagaagttg gtcgtgaggc actgggcagg taagtatcaa ggttacaaga     660
   caggtttaag gagaccaata gaaactgggc ttgtcgagac agagaagact cttgcgtttc     720
   tgataggcac ctattggtct tactgacatc cactttgcct ttctctccac aggtgtccac     780
   tcccaggttc aattacagct cttaagcagc cgcaagcttg atatcgaatt cctgcagccc     840
   gggggatcca ctagttctag agcggccgcc accgcggtgg agctcgaatt atcagatcga     900
   ttaataacta tgctcaaaaa ttgtgtacct ttagcttttt aatttgtaaa ggggttaata     960
   aggaatattt gatgtatagt gccttgacta gagatcataa tcagccatac cacatttgta    1020
   gaggttttac ttgctttaaa aaacctccca cacctccccc tgaacctgaa acataaaatg    1080
   aatgcaattg ttgttgttaa cttgtttatt gcagcttata atggttacaa ataaagcaat    1140
   agcatcacaa atttcacaaa taaagcattt ttttcactgc attctagttg tggtttgtcc    1200
   aaactcatca atgtatctta tcatgtctgg atcatcagat ctgccggtct ccctatagtg    1260
   agtcgtatta atttcgataa gccaggttaa cctgcattaa tgaatcggcc aacgcgcggg 1320
   gagaggcggt ttgcgtattg ggcgctcttc cgcttcctcg ctcactgact cgctgcgctc 1380
   ggtcgttcgg ctgcggcgag cggtatcagc tcactcaaag gcggtaatac ggttatccac 1440
   agaatcaggg gataacgcag gaaagaacat gtgagcaaaa ggccagcaaa aggccaggaa 1500
   ccgtaaaaag gccgcgttgc tggcgttttt ccataggctc cgcccccctg acgagcatca 1560
   caaaaatcga cgctcaagtc agaggtggcg aaacccgaca ggactataaa gataccaggc 1620
   gtttccccct ggaagctccc tcgtgcgctc tcctgttccg accctgccgc ttaccggata 1680
   cctgtccgcc tttctccctt cgggaagcgt ggcgctttct catagctcac gctgtaggta 1740
   tctcagttcg gtgtaggtcg ttcgctccaa gctgggctgt gtgcacgaac cccccgttca 1800
   gcccgaccgc tgcgccttat ccggtaacta tcgtcttgag tccaacccgg taagacacga 1860
   cttatcgcca ctggcagcag ccactggtaa caggattagc agagcgaggt atgtaggcgg 1920
   tgctacagag ttcttgaagt ggtggcctaa ctacggctac actagaagaa cagtatttgg 1980
   tatctgcgct ctgctgaagc cagttacctt cggaaaaaga gttggtagct cttgatccgg 2040
   caaacaaacc accgctggta gcggtggttt ttttgtttgc aagcagcaga ttacgcgcag 2100
   aaaaaaagga tctcaagaag atcctttgat cttttctacg gggtctgacg ctcagtggaa 2160
   cgaaaactca cgttaaggga ttttggtcat gggcgcgcct aggcttttgc aaagatcgat 2220
   caagagacag gatgaggatc gtttcgcagc ttttcattct gactgcaacg ggcaataagt 2280
   ctctgtgtgg attaaaaaaa gagtgtctga tagcagcttc tgaactggtt acctgccgtg 2340
   agtaaattaa aattttattg acttaggtca ctaaggcgcc ttgcgctgag gttgcgtcgt 2400
   gatatcatca gggcagaccg gttacatccc cctaacaagc tgtataaaga gaaatactat 2460
   ctcattggcg ttgcccgcac ctgacagtgc gacgttgggc tgcgtccgtc gaccaacggt 2520
   accgaggtaa cagcccaatc tatccatgat ctcggccagg ccgggtcggc cgttatgcag 2580
   cccggctcgg gtatgaagcc attaaggagc cgacccagcg cgaccgggcg gccggtcacg 2640
   ctgcctctgc tgaagcctgc ctgtcactcc ctgcgcggcg tacccgccgt tctcatcgag 2700
   taggctccgg atcgcgaccc cggacgggcc ctgggcccag gagcggccta tgacaaatgc 2760
   cgggtagcga tccggcattc agcattgact gcgcacggat ccagtccttg caggagcctt 2820
   atgccgaccg tagcaaaaaa tgagcccgag ccgatcgcga gttgtgatcc ggtcccgccg 2880
   attgccggtc gcgatgacgg tcctgtgtaa gcgttatcgt taccaattgt ttaagaagta 2940
   tatacgctac gaggtacttg ataacttctg cgtagcatac atgaggtttt gtataaaaat 3000
   ggcgggcgat atcaacgcag tgtcagaaat ccgaaacagt ctgcgggact ctggggttcg 3060
   aaatgaccga ccaagcgacg cccaacctgc catcacgaga tttcgattcc accgccgcct 3120
   tctatgaaag gttgggcttc ggaatcgttt tccgggacgc cggctggatg atcctccagc 3180
   gcggggatct catgctggag ttcttcgccc accctaggcg cgctcatgag cggatacata      3240
   tttgaatgta tttagaaaaa taaacaaata ggggttccgc gcacatttcc ccgaaaagtg      3300
   ccacctaaat tgtaagcgtt aatattttgt taaaattcgc gttaaatttt tgttaaatca      3360
   gctcattttt taaccaatag gccgaaatcg gcaaaatccc ttataaatca aaagaataga      3420
   ccgagatagg gttgagtgtt gttccagttt ggaacaagag tccactatta aagaacgtgg      3480
   actccaacgt caaagggcga aaaaccgtct atcagggcga tggcccacta cgtgaaccat      3540
   caccctaatc aagttttttg gggtcgaggt gccgtaaagc actaaatcgg aaccctaaag      3600
   ggagcccccg atttagagct tgacggggaa agccggcgaa cgtggcgaga aaggaaggga      3660
   agaaagcgaa aggagcgggc gctagggcgc tggcaagtgt agcggtcacg ctgcgcgtaa      3720
   ccaccacacc cgccgcgctt aatgcgccgc tacagggcgc gtcccattcg ccattcaggc      3780
   tgcgcaactg ttgggaaggg cgatcggtgc gggcctcttc gctattacgc cagctggcga      3840
   aagggggatg tgctgcaagg cgattaagtt gggtaacgcc agggttttcc cagtcacgac      3900
   gttgtaaaac gacggccagt gagcgcgcgt aatacgactc actatagggc gaattgggta      3960
   ccgggccccc cctcgagcag gatctataca ttgaatcaat attggcaatt agccatatta      4020
   gtcattggtt atatagcata aatcaatatt ggctattggc cattgcatac gttgtatcta      4080
   tatcataata tgtacattta tattggctca tgtccaatat gaccgccatg ttgacattga      4140
   ttattgacta gttattaata gtaatcaatt acggggtcat tagttcatag cccatatatg      4200
   gagttccgcg ttacataact tacggtaaat ggcccgcctg gc                          4242
<210> 2
   <211> 4242
   <212> DNA
   <213> Artificial sequence
<220>
   <223> Nucleotide sequence of plasmid pMB75.6
<400> 2
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gcgcgtaata cgactcacta tagggcgaat tgggtaccgg 660
   gccccccctc gagcaggatc tatacattga atcaatattg gcaattagcc atattagtca 720
   ttggttatat agcataaatc aatattggct attggccatt gcatacgttg tatctatatc 780
   ataatatgta catttatatt ggctcatgtc caatatgacc gccatgttga cattgattat 840
   tgactagtta ttaatagtaa tcaattacgg ggtcattagt tcatagccca tatatggagt 900
   tccgcgttac ataacttacg gtaaatggcc cgcctggctg accgcccaac gacccccgcc 960
   cattgacgtc aataatgacg tatgttccca tagtaacgcc aatagggact ttccattgac 1020
   gtcaatgggt ggagtattta cggtaaactg cccacttggc agtacatcaa gtgtatcata 1080
   tgccaagtcc gccccctatt gacgtcaatg acggtaaatg gcccgcctgg cattatgccc 1140
   agtacatgac cttacgggac tttcctactt ggcagtacat ctacgtatta gtcatcgcta 1200
   ttaccatggt gatgcggttt tggcagtaca tcaatgggcg tggatagcgg tttgactcac 1260
   ggggatttcc aagtctccac cccattgacg tcaatgggag tttgttttgg caccaaaatc 1320
   aacgggactt tccaaaatgt cgtaacaact ccgccccatt gacgcaaatg ggcggtaggc 1380
   gtgtacggtg ggaggtctat ataagcagag ctcgtttagt gaaccgtcag atcgcctgga 1440
   gacgccatcc acgctgtttt gacctccata gaagacaccg ggaccgatcc agcctcccct 1500
   cgaagccgat ctgataacgg taccgataag ctggcggccg attaagctac agaagttggt 1560
   cgtgaggcac tgggcaggta agtatcaagg ttacaagaca ggtttaagga gaccaataga 1620
   aactgggctt gtcgagacag agaagactct tgcgtttctg ataggcacct attggtctta 1680
   ctgacatcca ctttgccttt ctctccacag gtgtccactc ccaggttcaa ttacagctct 1740
   taagcagccg caagcttgat atcgaattcc tgcagcccgg gggatccact agttctagag 1800
   cggccgccac cgcggtggag ctcgaattat cagatcgatt aataactatg ctcaaaaatt 1860
   gtgtaccttt agctttttaa tttgtaaagg ggttaataag gaatatttga tgtatagtgc 1920
   cttgactaga gatcataatc agccatacca catttgtaga ggttttactt gctttaaaaa 1980
   acctcccaca cctccccctg aacctgaaac ataaaatgaa tgcaattgtt gttgttaact 2040
   tgtttattgc agcttataat ggttacaaat aaagcaatag catcacaaat ttcacaaata 2100
   aagcattttt ttcactgcat tctagttgtg gtttgtccaa actcatcaat gtatcttatc 2160
   atgtctggat catcagatct gccggtctcc ctatagtgag tcgtattaat ttcgataagc 2220
   caggttaacc tgcattaatg aatcggccaa cgcgcgggga gaggcggttt gcgtattggg 2280
   cgctcttccg cttcctcgct cactgactcg ctgcgctcgg tcgttcggct gcggcgagcg 2340
   gtatcagctc actcaaaggc ggtaatacgg ttatccacag aatcagggga taacgcagga 2400
   aagaacatgt gagcaaaagg ccagcaaaag gccaggaacc gtaaaaaggc cgcgttgctg 2460
   gcgtttttcc ataggctccg cccccctgac gagcatcaca aaaatcgacg ctcaagtcag 2520
   aggtggcgaa acccgacagg actataaaga taccaggcgt ttccccctgg aagctccctc 2580
   gtgcgctctc ctgttccgac cctgccgctt accggatacc tgtccgcctt tctcccttcg 2640
   ggaagcgtgg cgctttctca tagctcacgc tgtaggtatc tcagttcggt gtaggtcgtt 2700
   cgctccaagc tgggctgtgt gcacgaaccc cccgttcagc ccgaccgctg cgccttatcc 2760
   ggtaactatc gtcttgagtc caacccggta agacacgact tatcgccact ggcagcagcc 2820
   actggtaaca ggattagcag agcgaggtat gtaggcggtg ctacagagtt cttgaagtgg 2880
   tggcctaact acggctacac tagaagaaca gtatttggta tctgcgctct gctgaagcca 2940
   gttaccttcg gaaaaagagt tggtagctct tgatccggca aacaaaccac cgctggtagc 3000
   ggtggttttt ttgtttgcaa gcagcagatt acgcgcagaa aaaaaggatc tcaagaagat 3060
   cctttgatct tttctacggg gtctgacgct cagtggaacg aaaactcacg ttaagggatt 3120
   ttggtcatga gcgcgcctag gcttttgcaa agatcgatca agagacagga tgaggatcgt 3180
   ttcgcatgat tgaacaagat ggattgcacg caggttctcc ggccgcttgg gtggagaggc 3240
   tattcggcta tgactgggca caacagacaa tcggctgctc tgatgccgcc gtgttccggc 3300
   tgtcagcgca ggggcgcccg gttctttttg tcaagaccga cctgtccggt gccctgaatg 3360
   aactgcaaga cgaggcagcg cggctatcgt ggctggccac gacgggcgtt ccttgcgcag 3420
   ctgtgctcga cgttgtcact gaagcgggaa gggactggct gctattgggc gaagtgccgg 3480
   ggcaggatct cctgtcatct caccttgctc ctgccgagaa agtatccatc atggctgatg 3540
   caatgcggcg gctgcatacg cttgatccgg ctacctgccc attcgaccac caagcgaaac 3600
   atcgcatcga gcgagcacgt actcggatgg aagccggtct tgtcgatcag gatgatctgg 3660
   acgaagagca tcaggggctc gcgccagccg aactgttcgc caggctcaag gcgagcatgc 3720
   ccgacggcga ggatctcgtc gtgacccatg gcgatgcctg cttgccgaat atcatggtgg 3780
   aaaatggccg cttttctgga ttcatcgact gtggccggct gggtgtggcg gaccgctatc 3840
   aggacatagc gttggctacc cgtgatattg ctgaagagct tggcggcgaa tgggctgacc 3900
   gcttcctcgt gctttacggt atcgccgctc ccgattcgca gcgcatcgcc ttctatcgcc 3960
   ttcttgacga gttcttctga gcgggactct ggggttcgaa atgaccgacc aagcgacgcc 4020
   caacctgcca tcacgagatt tcgattccac cgccgccttc tatgaaaggt tgggcttcgg 4080
   aatcgttttc cgggacgccg gctggatgat cctccagcgc ggggatctca tgctggagtt 4140
   cttcgcccac cctaggcgcg ctcatgagcg gatacatatt tgaatgtatt tagaaaaata 4200
   aacaaatagg ggttccgcgc acatttcccc gaaaagtgcc ac 4242
<210> 3
   <211> 4242
   <212> DNA
   <213> Artificial sequence
<220>
   <223> Nucleotide sequence of plasmid pMB75.6_AscI
<400> 3
   tgaccgccca acgacccccg cccattgacg tcaataatga cgtatgttcc catagtaacg      60
   ccaataggga ctttccattg acgtcaatgg gtggagtatt tacggtaaac tgcccacttg     120
   gcagtacatc aagtgtatca tatgccaagt ccgcccccta ttgacgtcaa tgacggtaaa     180
   tggcccgcct ggcattatgc ccagtacatg accttacggg actttcctac ttggcagtac     240
   atctacgtat tagtcatcgc tattaccatg gtgatgcggt tttggcagta catcaatggg     300
   cgtggatagc ggtttgactc acggggattt ccaagtctcc accccattga cgtcaatggg     360
   agtttgtttt ggcaccaaaa tcaacgggac tttccaaaat gtcgtaacaa ctccgcccca     420
   ttgacgcaaa tgggcggtag gcgtgtacgg tgggaggtct atataagcag agctcgttta     480
   gtgaaccgtc agatcgcctg gagacgccat ccacgctgtt ttgacctcca tagaagacac     540
   cgggaccgat ccagcctccc ctcgaagccg atctgataac ggtaccgata agctggcggc     600
   cgattaagct acagaagttg gtcgtgaggc actgggcagg taagtatcaa ggttacaaga     660
   caggtttaag gagaccaata gaaactgggc ttgtcgagac agagaagact cttgcgtttc     720
   tgataggcac ctattggtct tactgacatc cactttgcct ttctctccac aggtgtccac     780
   tcccaggttc aattacagct cttaagcagc cgcaagcttg atatcgaatt cctgcagccc     840
   gggggatcca ctagttctag agcggccgcc accgcggtgg agctcgaatt atcagatcga     900
   ttaataacta tgctcaaaaa ttgtgtacct ttagcttttt aatttgtaaa ggggttaata     960
   aggaatattt gatgtatagt gccttgacta gagatcataa tcagccatac cacatttgta    1020
   gaggttttac ttgctttaaa aaacctccca cacctccccc tgaacctgaa acataaaatg    1080
   aatgcaattg ttgttgttaa cttgtttatt gcagcttata atggttacaa ataaagcaat    1140
   agcatcacaa atttcacaaa taaagcattt ttttcactgc attctagttg tggtttgtcc    1200
   aaactcatca atgtatctta tcatgtctgg atcatcagat ctgccggtct ccctatagtg    1260
   agtcgtatta atttcgataa gccaggttaa cctgcattaa tgaatcggcc aacgcgcggg    1320
   gagaggcggt ttgcgtattg ggcgctcttc cgcttcctcg ctcactgact cgctgcgctc    1380
   ggtcgttcgg ctgcggcgag cggtatcagc tcactcaaag gcggtaatac ggttatccac    1440
   agaatcaggg gataacgcag gaaagaacat gtgagcaaaa ggccagcaaa aggccaggaa    1500
   ccgtaaaaag gccgcgttgc tggcgttttt ccataggctc cgcccccctg acgagcatca    1560
   caaaaatcga cgctcaagtc agaggtggcg aaacccgaca ggactataaa gataccaggc    1620
   gtttccccct ggaagctccc tcgtgcgctc tcctgttccg accctgccgc ttaccggata 1680
   cctgtccgcc tttctccctt cgggaagcgt ggcgctttct catagctcac gctgtaggta 1740
   tctcagttcg gtgtaggtcg ttcgctccaa gctgggctgt gtgcacgaac cccccgttca 1800
   gcccgaccgc tgcgccttat ccggtaacta tcgtcttgag tccaacccgg taagacacga 1860
   cttatcgcca ctggcagcag ccactggtaa caggattagc agagcgaggt atgtaggcgg 1920
   tgctacagag ttcttgaagt ggtggcctaa ctacggctac actagaagaa cagtatttgg 1980
   tatctgcgct ctgctgaagc cagttacctt cggaaaaaga gttggtagct cttgatccgg 2040
   caaacaaacc accgctggta gcggtggttt ttttgtttgc aagcagcaga ttacgcgcag 2100
   aaaaaaagga tctcaagaag atcctttgat cttttctacg gggtctgacg ctcagtggaa 2160
   cgaaaactca cgttaaggga ttttggtcat gggcgcgcct aggcttttgc aaagatcgat 2220
   caagagacag gatgaggatc gtttcgcatg attgaacaag atggattgca cgcaggttct 2280
   ccggccgctt gggtggagag gctattcggc tatgactggg cacaacagac aatcggctgc 2340
   tctgatgccg ccgtgttccg gctgtcagcg caggggcgcc cggttctttt tgtcaagacc 2400
   gacctgtccg gtgccctgaa tgaactgcaa gacgaggcag cgcggctatc gtggctggcc 2460
   acgacgggcg ttccttgcgc agctgtgctc gacgttgtca ctgaagcggg aagggactgg 2520
   ctgctattgg gcgaagtgcc ggggcaggat ctcctgtcat ctcaccttgc tcctgccgag 2580
   aaagtatcca tcatggctga tgcaatgcgg cggctgcata cgcttgatcc ggctacctgc 2640
   ccattcgacc accaagcgaa acatcgcatc gagcgagcac gtactcggat ggaagccggt 2700
   cttgtcgatc aggatgatct ggacgaagag catcaggggc tcgcgccagc cgaactgttc 2760
   gccaggctca aggcgagcat gcccgacggc gaggatctcg tcgtgaccca tggcgatgcc 2820
   tgcttgccga atatcatggt ggaaaatggc cgcttttctg gattcatcga ctgtggccgg 2880
   ctgggtgtgg cggaccgcta tcaggacata gcgttggcta cccgtgatat tgctgaagag 2940
   cttggcggcg aatgggctga ccgcttcctc gtgctttacg gtatcgccgc tcccgattcg 3000
   cagcgcatcg ccttctatcg ccttcttgac gagttcttct gagcgggact ctggggttcg 3060
   aaatgaccga ccaagcgacg cccaacctgc catcacgaga tttcgattcc accgccgcct 3120
   tctatgaaag gttgggcttc ggaatcgttt tccgggacgc cggctggatg atcctccagc 3180
   gcggggatct catgctggag ttcttcgccc accctaggcg cgctcatgag cggatacata 3240
   tttgaatgta tttagaaaaa taaacaaata ggggttccgc gcacatttcc ccgaaaagtg 3300
   ccacctaaat tgtaagcgtt aatattttgt taaaattcgc gttaaatttt tgttaaatca 3360
   gctcattttt taaccaatag gccgaaatcg gcaaaatccc ttataaatca aaagaataga 3420
   ccgagatagg gttgagtgtt gttccagttt ggaacaagag tccactatta aagaacgtgg 3480
   actccaacgt caaagggcga aaaaccgtct atcagggcga tggcccacta cgtgaaccat 3540
   caccctaatc aagttttttg gggtcgaggt gccgtaaagc actaaatcgg aaccctaaag 3600
   ggagcccccg atttagagct tgacggggaa agccggcgaa cgtggcgaga aaggaaggga 3660
   agaaagcgaa aggagcgggc gctagggcgc tggcaagtgt agcggtcacg ctgcgcgtaa 3720
   ccaccacacc cgccgcgctt aatgcgccgc tacagggcgc gtcccattcg ccattcaggc 3780
   tgcgcaactg ttgggaaggg cgatcggtgc gggcctcttc gctattacgc cagctggcga 3840
   aagggggatg tgctgcaagg cgattaagtt gggtaacgcc agggttttcc cagtcacgac 3900
   gttgtaaaac gacggccagt gagcgcgcgt aatacgactc actatagggc gaattgggta 3960
   ccgggccccc cctcgagcag gatctataca ttgaatcaat attggcaatt agccatatta 4020
   gtcattggtt atatagcata aatcaatatt ggctattggc cattgcatac gttgtatcta 4080
   tatcataata tgtacattta tattggctca tgtccaatat gaccgccatg ttgacattga 4140
   ttattgacta gttattaata gtaatcaatt acggggtcat tagttcatag cccatatatg 4200
   gagttccgcg ttacataact tacggtaaat ggcccgcctg gc 4242
<210> 4
   <211> 4242
   <212> DNA
   <213> Artificial sequence
<220>
   <223> Nucelotide sequence of plasmid pLacZMB75.6
<400> 4
   tgaccgccca acgacccccg cccattgacg tcaataatga cgtatgttcc catagtaacg      60
   ccaataggga ctttccattg acgtcaatgg gtggagtatt tacggtaaac tgcccacttg     120
   gcagtacatc aagtgtatca tatgccaagt ccgcccccta ttgacgtcaa tgacggtaaa     180
   tggcccgcct ggcattatgc ccagtacatg accttacggg actttcctac ttggcagtac     240
   atctacgtat tagtcatcgc tattaccatg gtgatgcggt tttggcagta catcaatggg     300
   cgtggatagc ggtttgactc acggggattt ccaagtctcc accccattga cgtcaatggg     360
   agtttgtttt ggcaccaaaa tcaacgggac tttccaaaat gtcgtaacaa ctccgcccca     420
   ttgacgcaaa tgggcggtag gcgtgtacgg tgggaggtct atataagcag agctcgttta     480
   gtgaaccgtc agatcgcctg gagacgccat ccacgctgtt ttgacctcca tagaagacac     540
   cgggaccgat ccagcctccc ctcgaagccg atctgataac ggtaccgata agctggcggc     600
   cgattaagct acagaagttg gtcgtgaggc actgggcagg taagtatcaa ggttacaaga     660
   caggtttaag gagaccaata gaaactgggc ttgtcgagac agagaagact cttgcgtttc     720
   tgataggcac ctattggtct tactgacatc cactttgcct ttctctccac aggtgtccac     780
   tcccaggttc aattacagct cttaagcagc cgccaaaaca aaattcctca aaaatcatca     840
   tcgaatgaat ggtgaaataa tttccctgaa taactgtagt gttttcaggg cgcggcataa 900
   taattaacta tgctcaaaaa ttgtgtacct ttagcttttt aatttgtaaa ggggttaata 960
   aggaatattt gatgtatagt gccttgacta gagatcataa tcagccatac cacatttgta 1020
   gaggttttac ttgctttaaa aaacctccca cacctccccc tgaacctgaa acataaaatg 1080
   aatgcaattg ttgttgttaa cttgtttatt gcagcttata atggttacaa ataaagcaat 1140
   agcatcacaa atttcacaaa taaagcattt ttttcactgc attctagttg tggtttgtcc 1200
   aaactcatca atgtatctta tcatgtctgg atcatcagat ctgccggtct ccctatagtg 1260
   agtcgtatta atttcgataa gccaggttaa cctgcattaa tgaatcggcc aacgcgcggg 1320
   gagaggcggt ttgcgtattg ggcgctcttc cgcttcctcg ctcactgact cgctgcgctc 1380
   ggtcgttcgg ctgcggcgag cggtatcagc tcactcaaag gcggtaatac ggttatccac 1440
   agaatcaggg gataacgcag gaaagaacat gtgagcaaaa ggccagcaaa aggccaggaa 1500
   ccgtaaaaag gccgcgttgc tggcgttttt ccataggctc cgcccccctg acgagcatca 1560
   caaaaatcga cgctcaagtc agaggtggcg aaacccgaca ggactataaa gataccaggc 1620
   gtttccccct ggaagctccc tcgtgcgctc tcctgttccg accctgccgc ttaccggata 1680
   cctgtccgcc tttctccctt cgggaagcgt ggcgctttct catagctcac gctgtaggta 1740
   tctcagttcg gtgtaggtcg ttcgctccaa gctgggctgt gtgcacgaac cccccgttca 1800
   gcccgaccgc tgcgccttat ccggtaacta tcgtcttgag tccaacccgg taagacacga 1860
   cttatcgcca ctggcagcag ccactggtaa caggattagc agagcgaggt atgtaggcgg 1920
   tgctacagag ttcttgaagt ggtggcctaa ctacggctac actagaagaa cagtatttgg 1980
   tatctgcgct ctgctgaagc cagttacctt cggaaaaaga gttggtagct cttgatccgg 2040
   caaacaaacc accgctggta gcggtggttt ttttgtttgc aagcagcaga ttacgcgcag 2100
   aaaaaaagga tctcaagaag atcctttgat cttttctacg gggtctgacg ctcagtggaa 2160
   cgaaaactca cgttaaggga ttttggtcat gggcgcgcct aggcttttgc aaagatcgat 2220
   caagagacag gatgaggatc gtttcgcagc ttttcattct gactgcaacg ggcaataagt 2280
   ctctgtgtgg attaaaaaaa gagtgtctga tagcagcttc tgaactggtt acctgccgtg 2340
   agtaaattaa aattttattg acttaggtca ctaaggcgcc ttgcgctgag gttgcgtcgt 2400
   gatatcatca gggcagaccg gttacatccc cctaacaagc tgtataaaga gaaatactat 2460
   ctcattggcg ttgcccgcac ctgacagtgc gacgttgggc tgcgtccgtc gaccaacggt 2520
   accgaggtaa cagcccaatc tatccatgat ctcggccagg ccgggtcggc cgttatgcag 2580
   cccggctcgg gtatgaagcc attaaggagc cgacccagcg cgaccgggcg gccggtcacg 2640
   ctgcctctgc tgaagcctgc ctgtcactcc ctgcgcggcg tacccgccgt tctcatcgag 2700
   taggctccgg atcgcgaccc cggacgggcc ctgggcccag gagcggccta tgacaaatgc 2760
   cgggtagcga tccggcattc agcattgact gcgcacggat ccagtccttg caggagcctt 2820
   atgccgaccg tagcaaaaaa tgagcccgag ccgatcgcga gttgtgatcc ggtcccgccg 2880
   attgccggtc gcgatgacgg tcctgtgtaa gcgttatcgt taccaattgt ttaagaagta 2940
   tatacgctac gaggtacttg ataacttctg cgtagcatac atgaggtttt gtataaaaat 3000
   ggcgggcgat atcaacgcag tgtcagaaat ccgaaacagt ctgcgggact ctggggttcg 3060
   aaatgaccga ccaagcgacg cccaacctgc catcacgaga tttcgattcc accgccgcct 3120
   tctatgaaag gttgggcttc ggaatcgttt tccgggacgc cggctggatg atcctccagc 3180
   gcggggatct catgctggag ttcttcgccc accctaggcg cgctcatgag cggatacata 3240
   tttgaatgta tttagaaaaa taaacaaata ggggttccgc gcacatttcc ccgaaaagtg 3300
   ccacctaaat tgtaagcgtt aatattttgt taaaattcgc gttaaatttt tgttaaatca 3360
   gctcattttt taaccaatag gccgaaatcg gcaaaatccc ttataaatca aaagaataga 3420
   ccgagatagg gttgagtgtt gttccagttt ggaacaagag tccactatta aagaacgtgg 3480
   actccaacgt caaagggcga aaaaccgtct atcagggcga tggcccacta cgtgaaccat 3540
   caccctaatc aagttttttg gggtcgaggt gccgtaaagc actaaatcgg aaccctaaag 3600
   ggagcccccg atttagagct tgacggggaa agccggcgaa cgtggcgaga aaggaaggga 3660
   agaaagcgaa aggagcgggc gctagggcgc tggcaagtgt agcggtcacg ctgcgcgtaa 3720
   ccaccacacc cgccgcgctt aatgcgccgc tacagggcgc gtcccattcg ccattcaggc 3780
   tgcgcaactg ttgggaaggg cgatcggtgc gggcctcttc gctattacgc cagctggcga 3840
   aagggggatg tgctgcaagg cgattaagtt gggtaacgcc agggttttcc cagtcacgac 3900
   gttgtaaaac gacggccagt gagcgcgcgt aatacgactc actatagggc gaattgggta 3960
   ccgggccccc cctcgaggtc gacggtatcg ataagcttga tatcgaattc ctgcagcccg 4020
   ggggatccac tagttctaga gcggccgcca ccgcggtgga gctccagctt ttgttccctt 4080
   tagtgagggt taattgcgcg cttggcgtaa tcatggtcat agctgtttcc tgtgtgaaat 4140
   tgttatccgc tcacaattcc acacaacata cgagccggaa gcataaagtg taaagcctgg 4200
   ggtgcctaat gagtgagcta actcacatta attgcgttgc gc 4242
<210> 5
   <211> 20
   <212> DNA
   <213> Artificial sequence
<220>
   <223> Primer
<400> 5
   gtgcgcggaa cccctatttg 20
<210> 6
   <211> 20
   <212> DNA
   <213> Artificial sequence
<220>
   <223> Primer
<400> 6
   gcgtacccgc cgttctcatc 20
<210> 7
   <211> 20
   <212> DNA
   <213> Artificial sequence
<220>
   <223> Primer
<400> 7
   gtctgacgct cagtggaacg 20
<210> 8
   <211> 22
   <212> DNA
   <213> Artificial sequence
<220>
   <223> Primer
<400> 8
   gttccagttt ggaacaagag tc 22
<210> 9
   <211> 20
   <212> DNA
   <213> Artificial sequence
<220>
   <223> Primer
<400> 9
   ggcaattagc catattagtc 20
<210> 10
   <211> 22
   <212> DNA
   <213> Artificial sequence
<220>
   <223> Primer
<400> 10
   gcagagctcg tttagtgaac cg 22
<210> 11
   <211> 21
   <212> DNA
   <213> Artificial sequence
<220>
   <223> Primer
<400> 11
   gatcataatc agccatacca c 21
<210> 12
   <211> 27
   <212> DNA
   <213> Artificial sequence
<220>
   <223> Primer
<400> 12
   cgtcttgagt ccaacccggt aagacac 27
<210> 13
   <211> 24
   <212> DNA
   <213> Artificial sequence
<220>
   <223> Primer
<400> 13
   ccacaggtgt ccactcccag gttc 24
<210> 14
   <211> 21
   <212> DNA
   <213> Artificial sequence
<220>
   <223> Primer
<400> 14
   ctagtcaagg cactatacat c 21
<210> 15
   <211> 22
   <212> DNA
   <213> Artificial sequence
<220>
   <223> Primer
<400> 15
   tccacagaat caggggataa cg 22
<210> 16
   <211> 23
   <212> DNA
   <213> Artificial sequence
<220>
   <223> Primer
<400> 16
   gctcactcat taggcacccc agg 23
<210> 17
   <211> 22
   <212> DNA
   <213> Artificial sequence
<220>
   <223> Primer
<400> 17
   cgcgtaatac gactcactat ag 22

## Description

### FIELD OF THE INVENTION

The present invention generally relates to an immunomodulator composition for use with an immunogen to prevent infection or to improve an immune response to a vaccine antigen of in fish. The document WO2012032497 describes gene vaccination liposomes suitable for oral vaccination of aquatic animals.

### SUMMARY OF THE INVENTION

The invention is as defined in the claims.

The present invention relates to methods of using immunostimulatory plasmids with immunogens or vaccine antigens and a liposome delivery vehicle to modulate immunity in fish. The immunostimulatory plasmid comprises a nucleic acid sequence having at least 80% sequence identity with the sequence of SEQ ID NO: 1, SEQ ID NO: 2, SEQ ID NO: 3, SEQ ID NO: 4. In some aspects, the immunostimulatory plasmid may comprise a nucleic acid molecule having at least 84% sequence identity with the sequence of SEQ ID NO: 4. In some aspects, the immunostimulatory plasmid may comprise the sequence of SEQ ID NO: 1. In some aspects, the immunostimulatory plasmid may comprise the sequence of SEQ ID NO: 4. In some aspects, the immunostimulatory plasmid may comprise the sequence of SEQ ID NO: 2. In some aspects, the immunostimulatory plasmid may comprise the sequence of SEQ ID NO: 3.

In other aspects, the immunostimulatory plasmid may consist of a nucleic acid sequence having at least 80% sequence identity with the sequence of SEQ ID NO: 1, SEQ ID NO: 2, SEQ ID NO: 3, SEQ ID NO: 4. In some aspects, the immunostimulatory plasmid may consist of a nucleic acid molecule having at least 84% sequence identity with the sequence of SEQ ID NO: 4. In some aspects, the immunostimulatory plasmid may consist of the sequence of SEQ ID NO: 1. In some aspects, the immunostimulatory plasmid may consist of the sequence of SEQ ID NO: 4. In some aspects, the immunostimulatory plasmid may consist of the sequence of SEQ ID NO: 2. In some aspects, the immunostimulatory plasmid may consist of the sequence of SEQ ID NO: 3.

In some aspects, the immunostimulatory plasmid preferably does not comprise a nucleic acid sequence encoding a full-length or functional selectable or screenable marker. In other aspects, the immunostimulatory plasmid comprises a nucleic acid sequence encoding a selectable or screenable marker that is not an antibiotic resistance gene.

Pharmaceutical formulations comprising any of the immunostimulatory plasmids, or DNA sequences, and a pharmaceutically acceptable carrier are disclosed herein.

The immunomodulator compositions according to the present invention also comprise a liposome delivery vehicle, as defined in the claims.

Other objects and features will be in part apparent and in part pointed out hereinafter.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

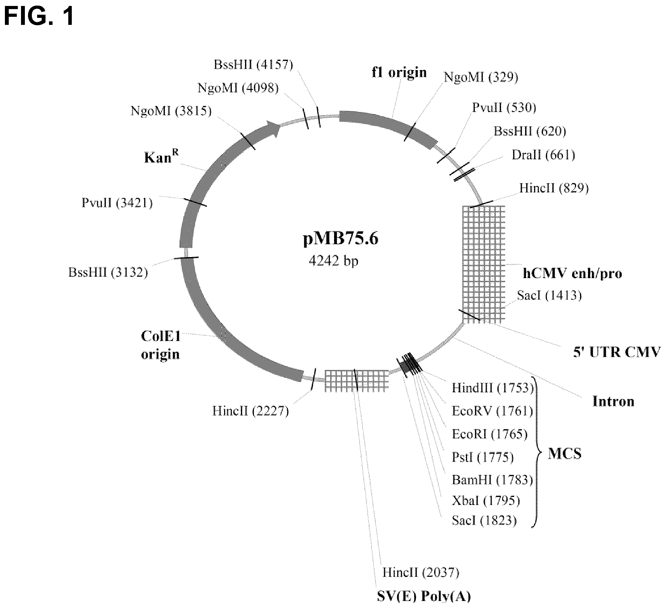

**FIG. 1** shows a map of the pMB75.6 plasmid (SEQ ID NO: 2);

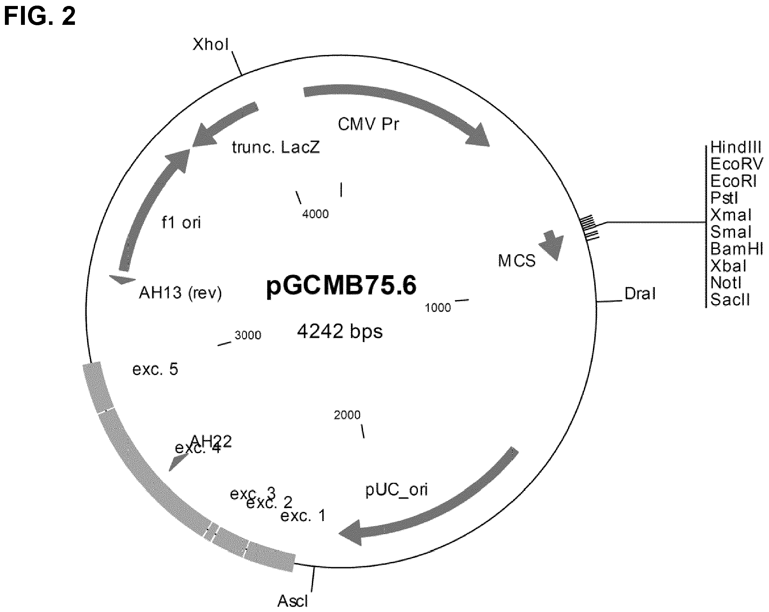

**FIG. 2** shows a map of the pGCMB75.6 plasmid (SEQ ID NO: 1);
**FIG. 3** shows a map of the pLacZMB75.6 plasmid (SEQ ID NO: 4);
**FIG. 4** graphically illustrates the daily mortality percentage post-challenge for fish tank M5.
**FIG. 5** graphically illustrates the daily mortality percentage post-challenge for fish tank M6.
**FIG. 6** shows Kaplan-Meier survival curves for combined mortalities from each group of the vaccine study. Vertical axis is the probability of the survival at a specific time DPC (horizontal axis). Crosses at the end of the curve represent censored data (fish alive beyond 27 DPC). DPC: days post challenge.
**FIG. 7** depicts a pair-wise comparison between the 12 groups of fish from the vaccine study, with linked groups (represented as nodes displayed by the green circles) showing no significant difference between them (α=0.05).
**FIG. 8** graphically illustrates the relative percentage of survival for vaccine study groups when compared with the dextrose 5%-treated control group. Comparisons were made between the cumulative mortality at the end of experiment (RPSend). Significant cumulative mortalities differences (α = 0.05) between the dextrose 5 % group and the other treatments are indicated with*.

### DETAILED DESCRIPTION OF THE INVENTION

The invention is as defined in the claims.

In accordance with the present invention, a composition for use in eliciting an immune response in fish has been discovered. It has been discovered that such immunomodulator compositions may be used to modulate the immune system of fish. The invention is particularly useful in the treatment and prevention of infectious diseases caused by microorganisms, such as, without limitation, viruses, bacteria, mold, fungus, yeast, parasites and other microbes known in the art. The methods of using the immunomodulator compositions are discussed in more detail below.

### I. Compositions

Compositions described herein, are generally able to be used as a prophylactic therapy, metaphylactic therapy, or treatment therapy for infectious diseases. Such compositions are referred to herein as immunomodulator compositions. The immunomodulator compositions include at least an immunostimulatory plasmid, or immunostimulatory DNA sequence, capable of inducing an immune response in fish and include a liposome delivery vehicle, as recited in the claims.

### A. Nucleic Acids

The nucleic acid molecules described herein are enriched in CpG motifs. Such CpG motifs may induce immune stimulation via immune surveillance receptors including specific Toll-like receptors, such as TLR9 and TLR21. In addition the nucleic acid molecules described herein also contain non-CpG immunostimulatory motifs. The nucleic acid molecules contain about 2-20% CpG motifs over the frequency of CpG motifs expected in random nucleic acid sequences. According to one aspect of the present invention to the immunostimulatory plasmid is the pGCMB75.6 plasmid described herein which does not comprise any full-length or functional selectable or screenable marker genes. The sequence of pGCMB75.6 is provided in SEQ ID NO: 1.

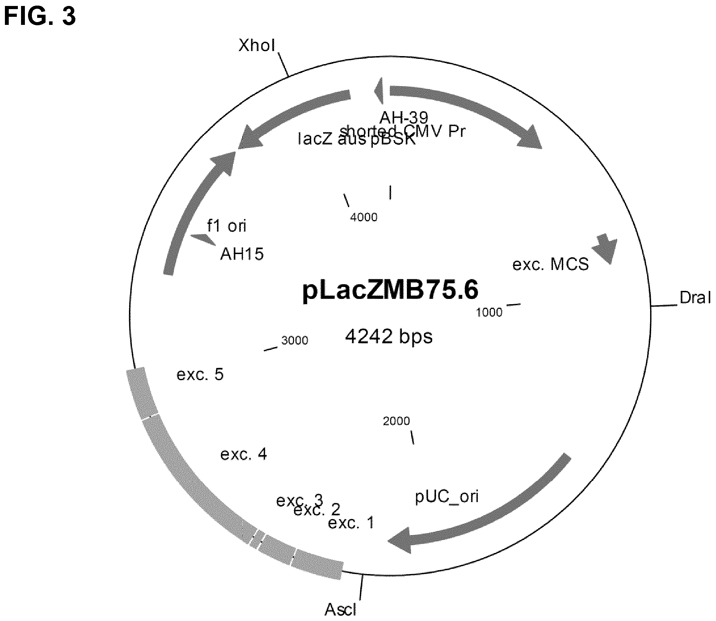

According to another aspect of the present invention, the immunostimulatory plasmid comprises a nucleic acid sequence coding for a selectable or screenable marker gene that is not an antibiotic resistance gene. For example, the pLacZMB75.6 plasmid described herein comprises a LacZ gene as a screenable marker. A map of pLacZMB75.6 is provided in FIG. 3 and the nucleotide sequence of pLacZMB75.6 is provided as SEQ ID NO: 4. As shown in FIG. 3, pLacZMB75.6 is similar to pGCMB75.6, but contains a LacZ screenable marker.

It will be appreciated that the nucleotide sequences of the pMB75.6 (SEQ ID NO: 2 OR SEQ ID NO: 3 (p MB75.6 with an AscI restriction site)), pGCMB75.6 (SEQ ID NO: 1) or pLacZMB75.6 (SEQ ID NO: 4) plasmids according to the present invention may be varied to a certain extent without significantly adversely affecting their immunostimulatory properties. In some aspects, the present invention relates to an immunostimulatory plasmid comprising a nucleic acid sequence having at least 80% sequence identity with the sequence of pGCMB75.6 (SEQ ID NO: 1). The immunostimulatory plasmid preferably comprises a nucleic acid sequence having at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with the sequence of pGCMB75.6 (SEQ ID NO: 1). In some aspects, the immunostimulatory plasmid more preferably comprises the sequence of pGCMB75.6 (SEQ ID NO: 1).

In some aspects, the present invention relates to an immunostimulatory plasmid comprising a nucleic acid sequence having at least 80% sequence identity with the sequence of pLacZMB75.6 (SEQ ID NO: 4). The immunostimulatory plasmid preferably comprises a nucleic acid sequence having at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with the sequence of pLacZMB75.6 (SEQ ID NO: 4). In some aspects, the immunostimulatory plasmid more preferably comprises the sequence of pLacZMB75.6 (SEQ ID NO: 4).

In some aspects, the present invention relates to an immunostimulatory plasmid comprising a nucleic acid sequence having at least 80% sequence identity with the sequence of SEQ ID NO: 2. The immunostimulatory plasmid preferably comprises a nucleic acid sequence having at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with the sequence of SEQ ID NO: 2. In some aspects, the immunostimulatory plasmid more preferably comprises the sequence of SEQ ID NO: 2.

In some aspects, the present invention relates to an immunostimulatory plasmid comprising a nucleic acid sequence having at least 80% sequence identity with the sequence of SEQ ID NO: 3. The immunostimulatory plasmid preferably comprises a nucleic acid sequence having at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with the sequence of SEQ ID NO: 3. In some aspects, the immunostimulatory plasmid more preferably comprises the sequence of SEQ ID NO: 3.

In some aspects, the present invention relates to an immunostimulatory plasmid consisting of a nucleic acid sequence having at least 80% sequence identity with the sequence of pGCMB75.6 (SEQ ID NO: 1). The immunostimulatory plasmid preferably consists of a nucleic acid sequence having at least 81%, at least 82%, at least 83%, at least 84%, at least, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with the sequence of pGCMB75.6 (SEQ ID NO: 1). In some aspects, the immunostimulatory plasmid more preferably consists of the sequence of pGCMB75.6 (SEQ ID NO: 1).

In some aspects, the present invention relates to an immunostimulatory plasmid consisting of a nucleic acid sequence having at least 80% sequence identity with the sequence of pLacZMB75.6 (SEQ ID NO: 4). The immunostimulatory plasmid preferably consists of a nucleic acid sequence having at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with the sequence of pLacZMB75.6 (SEQ ID NO: 4). In some aspects, the immunostimulatory plasmid more preferably consists of the sequence of pLacZMB75.6 (SEQ ID NO: 4).

In some aspects, the present invention relates to an immunostimulatory plasmid consisting of a nucleic acid sequence having at least 80% sequence identity with the sequence of SEQ ID NO: 2. The immunostimulatory plasmid preferably consists of a nucleic acid sequence having at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with the sequence of SEQ ID NO: 2. In some aspects, the immunostimulatory plasmid more preferably consists of the sequence of SEQ ID NO: 2.

In some aspects, the present invention relates to an immunostimulatory plasmid consisting of a nucleic acid sequence having at least 80% sequence identity with the sequence of SEQ ID NO: 3. The immunostimulatory plasmid preferably consists of a nucleic acid sequence having at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with the sequence of SEQ ID NO: 3. In some aspects, the immunostimulatory plasmid more preferably consists of the sequence of SEQ ID NO: 3.

In some aspects, where the nucleotide sequence of the immunostimulatory plasmid varies from the sequences provided in SEQ ID NOs. 1, 2, 3, and 4 the CpG dinucleotides in the plasmid are preferably left intact. Alternatively, if the nucleotide sequence of the plasmid is altered such that a CpG dinucleotide is eliminated, the sequence of the plasmid may be altered at another location such that the total number of CpG dinucleotides in the plasmid remains the same. Further CpG dinucleotides in addition to those already present in the nucleotide sequences of pGCMB75.6 or pLacZMB75.6 may also be introduced into the plasmid. Thus, for example, the immunostimulatory plasmids described herein preferably comprise at least about 200, at least about 220, at least about 240, at least about 260, at least about 270, at least about 275, at least about 280, at least about 283, at least about 285, or at least about 288 CpG dinucleotides. For example, the immunostimulatory plasmid can comprise 283 CpG dinucleotides.

In some aspects, where the nucleotide sequence of the immunostimulatory plasmid varies from the sequences provided herein, the CpG motif types in the plasmid are varied to modulate the resultant activation of the cytosolic DNA surveillance molecules. For example, the number of immune stimulatory CpG motifs may be increased to increase the activation of specific cytosolic DNA surveillance molecules responsive to a specific threshold of immunostimulatory plasmid/DNA. By way of further example, the number of non-immune stimulatory CpG motifs may be increased to decrease the activation of specific cytosolic DNA surveillance molecules and/or increase activation of other DNA surveillance molecules.

In particular, the present disclosure relates to pharmaceutical formulations comprising any of the immunostimulatory plasmids or DNA sequences described herein and a pharmaceutically acceptable carrier.

### B. Immunomodulator

Suitable immunomodulator compositions for use with the immunostimulatory plasmids described herein are described in U.S. Patent Application Publications Nos. 2012/0064151 A1 and 2013/0295167 A1.

The immunomodulator composition comprises a liposome delivery vehicle and at least one of the immunostimulatory plasmids, or DNA sequences, described herein.

Liposome delivery vehicles according to the present invention have a polycationic lipid composition (i.e., cationic liposomes) and a cholesterol backbone conjugated to polyethylene glycol. Those liposome compositions are N-[1-(2,3-dioleyloxy)propyl]-N,N,N-trimethylammonium chloride (DOTMA) and cholesterol, N-[1-(2,3-dioleoyloxy)propyl]-N,N,N-trimethylammonium chloride (DOTAP) and cholesterol, 1-[2-(oleoyloxy)ethyl]-2-oleyl-3-(2-hydroxyethyl)-imidazolinium chloride (DOTIM) and cholesterol, dimethyldioctadecylammonium bromide (DDAB) and cholesterol. A most preferred liposome composition for use as a delivery vehicle includes DOTIM and cholesterol.

A suitable nucleic acid molecule includes any of the immunostimulatory plasmids described herein. Coding nucleic acid sequences encode at least a portion of a protein or peptide, while non-coding sequence does not encode any portion of a protein or peptide. According to the present invention, "non-coding" nucleic acids can include regulatory regions of a transcription unit, such as a promoter region. The term, "empty vector" can be used interchangeably with the term "non-coding," and particularly refers to a nucleic acid sequence in the absence of a protein coding portion, such as a plasmid vector without a gene insert. Expression of a protein encoded by the plasmids described herein is not required for inducing an immune response; therefore the plasmids need not contain any coding sequences operatively linked to a transcription control sequence. However, further advantages may be obtained (i.e., antigen-specific and enhanced immunity) by including in the composition nucleic acid sequence (DNA or RNA) which encodes an immunogen and/or a cytokine. Such a nucleic acid sequence encoding an immunogen and/or a cytokine may be included in the immunostimulatory plasmids described herein, or may be included in a separate nucleic acid (e.g., a separate plasmid) in the composition.

Complexing a liposome with the immunostimulatory plasmids, or immunostimulatory DNA sequence, described herein may be achieved using methods standard in the art or as described in U.S. Patent No. 6,693,086. A suitable concentration of a plasmid to add to a liposome includes a concentration effective for delivering a sufficient amount of the plasmid into a fish such that a systemic immune response is elicited. For example, from about 0.1 µg to about 10 µg of plasmid can be combined with about 8 nmol liposomes, from about 0.5 µg to about 5 µg of plasmid can be combined with about 8 nmol liposomes, or about 1.0 µg of plasmid can be combined with about 8 nmol liposomes. The ratio of plasmid to lipid (µg plasmid:nmol lipid) in a composition can be at least about 1:1 plasmid:lipid by weight (e.g., 1 µg plasmid: 1 nmol lipid). For example, the ratio of plasmid to lipids can be at least about 1:5, at least about 1:10, or at least about 1:20. Ratios expressed herein are based on the amount of cationic lipid in the composition, and not on the total amount of lipid in the composition. The ratio of plasmid to lipids in a composition of the invention is suitably from about 1:1 to about 1:80 plasmid:lipid by weight; from about 1:2 to about 1:40 plasmid:lipid by weight; from about 1:3 to about 1:30 plasmid: lipid by weight; or from about 1:6 to about 1:15 plasmid:lipid by weight.

### C. Biological Agent

Any of the immunomodulator compositions described herein further comprise at least one biological agent, in addition to the liposome delivery vehicle and at least one of the plasmids described herein.

Such biological agents include immunogens and vaccines. Suitable immunogens are proteins which elicit a humoral and/or cellular immune response such that administration of the immunogen to a fish mounts an immunogen-specific immune response against the same or similar proteins that are encountered within the tissues of the fish. An immunogen may include a pathogenic antigen expressed by a bacterium, a virus, a parasite or a fungus. Preferred antigens include antigens derived from organisms which cause an infectious disease in a fish. According to the present invention, an immunogen may be any portion of a protein, naturally occurring or synthetically derived, which elicits a humoral and/or cellular immune response. As such, the size of an antigen or immunogen may be as small as about 5-12 amino acids and as large as a full length protein, including any sizes in between. The antigen may be a multimer protein or fusion protein. The antigen may be a purified antigen. Alternatively, the immunogen can be encoded by the immunostimulatory plasmid or by another nucleic acid included in the immunomodulator composition. Where the immune enhancer protein or immunogen is encoded by a nucleic acid molecule in the immunomodulator composition, the nucleic acid sequence encoding the immune enhancer protein or immunogen is operatively linked to a transcription control sequence, such that the immunogen is expressed in a tissue of a fish, thereby eliciting an immunogen-specific immune response in the fish, in addition to the non-specific immune response. Techniques to screen for immunogenicity, such as pathogen antigen immunogenicity or cytokine activity are known to those of skill in the art and include a variety of *in vitro* and *in vivo* assays.

Where the biological agent is a vaccine, the vaccine may include a live, infectious, viral, bacterial, or parasite vaccine or a killed, inactivated, viral, bacterial, or parasite vaccine, or subunit vaccine, or any vaccine known in the art. One or more vaccines may be used in combination with the immunomodulator composition of the present invention. Suitable vaccines include those known in the art for aquatic species.

### II. Methods

An object of the present invention is to provide immunomodulator compositions with immunostimulatory plasmids (or DNA sequence), for methods that stimulate immunity and provide protective immunity to uninfected fish, protective immunity to infected fish, enhanced immunity to uninfected fish, enhanced immunity to infected fish, therapeutic immunity to infected fish, or combinations thereof. As such, the compositions of the invention are used to prophylactically immunize a fish or be used to treat a fish. The methods described herein include administrating an immunostimulatory plasmid, or DNA sequence, described herein to a fish, and stimulating the immune system in the fish.

### A. Methods of Stimulating the Immune System of a fish

Usually, the immunomodulator composition enhances the operation of at least one biological agent such as a vaccine, when administered prior to such a vaccine, co-administered with a vaccine, administered post vaccination, or mixed with the vaccine. In some aspects, the methods provide new treatment strategies for protecting recipient fish from infectious diseases and treating populations having infectious disease. In some aspects, the methods provide a more rapid, a longer and better protection against a disease when the immunomodulator is used in combination with a vaccine, compared to use of the vaccine without the immunomodulator composition.

An immune response can be activated in a recipient fish by administering an effective amount of an immunomodulator composition, which includes any of the liposome delivery vehicles described herein, any of the immunostimulatory plasmids (or DNA sequences) described herein, and any of the biological agents described herein. It is contemplated that the biological agent may be mixed with or co-administered with the immunomodulator or independently thereof. Independent administration may be prior to or after administration of the immunomodulator. It is also contemplated that more than one administration of the immunomodulator or biological agent may be used. Furthermore, more than one biological agent may be co-administered with the immunomodulator, administered prior to the immunomodulator, administered after administration of the immunomodulator, or concurrently with the immunomodulator.

### B. Methods of Improving Survivability of a fish

### C. Conditions for Use

The methods of the invention activate an immune response in the fish such that the fish is protected from a disease that is amenable to elicitation of an immune response. As used herein, the phrase "protected from a disease" refers to reducing the symptoms of the disease; reducing the occurrence of the disease; reducing the clinical or pathologic severity of the disease; or reducing shedding of a pathogen causing a disease. Protecting a fish can refer to the ability of a therapeutic composition of the present invention, when administered to a fish, to prevent a disease from occurring, cure, and/or alleviate or reduce disease symptoms, clinical signs, pathology, or causes. As such, protecting a fish from a disease encompasses both preventing disease occurrence (prophylactic treatment) and treating a fish that has a disease (therapeutic treatment). The term "disease" refers to any deviation from the normal health of a fish and includes a state when disease symptoms are present, as well as conditions in which a deviation (e.g., infection, gene mutation, genetic defect, etc.) has occurred, but symptoms are not yet manifested.

Methods of the invention may be used for the prevention of disease, stimulation of effector cell immunity against disease, elimination of disease, alleviation of disease, and prevention of a secondary disease resulting from the occurrence of a primary disease.

In some aspects, methods described herein may be used to improve the innate immune response of the fish when co-administered with a vaccine versus administration of the vaccine by itself. In some aspects, methods described herein may be used to improve the acquired immune response of the fish when co-administered with a vaccine versus administration of the vaccine by itself. Generally a vaccine once administered does not immediately protect the fish as it takes time to stimulate acquired immunity. The term "improve" refers, in the present invention, to elicitation of an innate immune response in the fish until the vaccine starts to protect the fish and/or to prolong the period of protection, via acquired immunity, given by the vaccine.

In some aspects, methods of the invention include administering the composition to protect against infection of a wide variety of pathogens. The composition administered may or may not include a specific antigen to elicit a specific response. It is contemplated that the methods of the invention will protect the recipient fish from disease resulting from infectious microbial agents including, without limitation, viruses, bacteria, fungi, and parasites. A skilled artisan will recognize and appreciate that a immunomodulator composition, as described herein, is effective against numerous infectious agents, which are too numerous to list. The infectious agents provided herein are provided for exemplary purposes and are provided without limitation of the scope of use.

### G. Administration

A variety of administration routes are available. The particular mode selected will depend, of course, upon the particular biological agents selected, the age and general health status of the fish, the particular condition being treated and the dosage required for therapeutic efficacy. The methods of this invention may be practiced using any mode of administration that produces effective levels of activation of an immune response without causing clinically unacceptable adverse effects. The compositions may conveniently be presented in unit dosage form and may be prepared by any of the methods well known in the art.

The immunomodulator composition may be administered intravenously, intramuscularly, intradermally, intraperitoneally, subcutaneously, by spray, in ovo, orally, intraocularly, intratracheally, intranasally, by immersion, uptake via skin or gills during bath, or by other methods known in the art. In one aspect, the immunomodulator is administered subcutaneously. In another aspect, the immunomodulator may be administered intramuscularly. In another aspect, the immunomodulator is administered as a spray. In another aspect, the immunomodulator may be administered orally. In another aspect, the immunomodulator may be administered subcutaneously.

In one respect, the immunomodulator may be administered by itself to the fish prior to challenge (or infection). In another aspect, the immunomodulator may be administered by itself to the fish post challenge (or infection). In another aspect, the immunomodulator may be administered by itself to the fish at the same time as challenge (or infection).

In some aspects, the immunomodulator composition may be co-administered at the same time as the vaccination prior to challenge. In some aspects, the immunomodulator composition may be co-administered at the same time as the vaccination at the same time as challenge (or infection). In some aspects, the co-administration may include administering the vaccine and immunomodulator in the same general location on the fish at two different sites next to each other (i.e., injections next to each other at the neck of the fish), on opposing sides of the fish at the same general location (i.e., one on each side of the neck), or on different locations of the same fish. In some aspects, the immunomodulator composition can be administered prior to vaccination and challenge. In some aspects, the immunomodulator composition may be administered after vaccination but prior to challenge. The immunomodulator composition can be administered after challenge to a fish that has been vaccinated prior to challenge (or infection).

A skilled artisan will recognize that administration routes may vary depending upon the fish and the health or state of the fish. The administration routes provided for are for exemplary purposes and are provided without limitation.

Vaccination may be performed at any age. The vaccine may be administered subcutaneously, by spray, orally, intraocularly, intratracheally, intranasally, *in ovo,* by immersion or by other methods know in the art. Oral vaccines may be administered in feed or water. Further, it is contemplated that the methods of the invention may be used based on routine vaccination schedules.

The immunomodulator composition may also be administered subcutaneously, by intraocularly, intratracheally, intranasally, *in ovo,* or by other methods known in the art. For example, the immunomodulator composition can be administered *in ovo.* Alternatively, the immunomodulator composition can be administered as a spray.

The administration to the egg may be prior to challenge (or infection) or post challenge.

The immunomodulator can be administered to a fish from about 1 to about 14 days prior to challenge or from about 1 to about 14 days post challenge. For example, the immunomodulator can be administered from about 1 to about 7 days prior to challenge or from about 1 to about 7 days post challenge. The immunomodulator is suitably administered 1, 2, 3, 4, 5, 6, 7 days prior to challenge or 1, 2, 3, 4, 5, 6, 7 days post challenge.

Other delivery systems may include time-release, delayed release, or sustained release delivery systems. Such systems can avoid repeated administrations of the compositions therefore increasing convenience. Many types of release delivery systems are available and known to those of ordinary skill in the art. They include polymer based systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent No. 5,075,109.

Delivery systems also include non-polymer systems that are lipids including sterols such as cholesterol, cholesterol esters, and fatty acids or neutral fats such as mono-, di-, and tri-glycerides; hydrogel release systems; silastic systems; peptide based systems; wax coatings; compressed tablets using conventional binders and excipients; partially fused implants; and the like. Specific examples include, but are not limited to, erosional systems in which an agent of the invention is contained in a form within a matrix such as those described in U.S. Patent Nos. 4,452,775, 4,675,189, and 5,736,152, and diffusional systems in which an active component permeates at a controlled rate from a polymer such as described in U.S. Patent Nos. 3, 854,480, 5,133,974, and 5,407,686. In addition, pump-based hardware delivery systems can be used, some of which are adapted for implantation.

As various changes could be made in the above composition, products, and methods without departing from the scope of the invention, it is intended that all matter contained in the above description and in the examples given below shall be interpreted as illustrative of the invention, which is as defined in the claims.

### DEFINITIONS

The term "effective amount" refers to the amount necessary or sufficient to realize a desired biologic effect. For example, an effective amount of immunomodulator for treating or preventing an infectious disease is that amount necessary to cause the development of an immune response upon exposure to the microbe, thus causing a reduction in the amount of microbe within the fish and preferably the eradication of the microbe. The effective amount for any particular application can vary depending on such factors as the disease or condition being treated, the size of the fish, or the severity of the disease or condition. One of ordinary skill in the art can empirically determine the effective amount of immunomodulator without necessitating undue experimentation.

The term "cytokine" refers to an immune enhancing protein family. The cytokine family includes hematopoietic growth factor, interleukins, interferons, immunoglobulin superfamily molecules, tumor necrosis factor family molecules and chemokines (i.e. proteins that regulate the migration and activation of cells, particularly phagocytic cells). Exemplary cytokines include, without limitation, interleukin-2 (IL-2), interleukin-12 (IL-12), interleukin-15 (IL-15), interleukin-18 (IL-18), interferon-α (IFNα), and interferon-y (IFNγ).

The term "elicit" can be used interchangeably with the terms activate, stimulate, generate or upregulate.

The term "eliciting an immune response" in a fish refers to specifically controlling or influencing the activity of the immune response, and can include activating an immune response, upregulating an immune response, enhancing an immune response and/or altering an immune response (such as by eliciting a type of immune response which in turn changes the prevalent type of immune response in a fish from one which is harmful or ineffective to one which is beneficial or protective).

The term "operatively linked" refers to linking a nucleic acid molecule to a transcription control sequence in a manner such that the molecule is able to be expressed when transfected (i.e., transformed, transduced or transfected) into a host cell. Transcriptional control sequences are sequences which control the initiation, elongation, and termination of transcription. Particularly important transcription control sequences are those which control transcription initiation, such as promoter, enhancer, operator and repressor sequences. A variety of such transcription control sequences are known to those skilled in the art. Preferred transcription control sequences include those which function in avian, fish, mammalian, bacteria, viral, plant, and insect cells. While any transcriptional control sequences may be used with the invention, the sequences may include naturally occurring transcription control sequences naturally associated with a sequence encoding an immunogen or immune stimulating protein.

The terms "nucleic acid molecule" and "nucleic acid sequence" can be used interchangeably and include DNA, RNA, or derivatives of either DNA or RNA. The terms also include oligonucleotides and larger sequences such as plasmids, such as the immunostimulatory plasmids described herein, and including both nucleic acid molecules that encode a protein or a fragment thereof, and nucleic acid molecules that comprise regulatory regions, introns, or other non-coding DNA or RNA. Typically, an oligonucleotide has a nucleic acid sequence from about 1 to about 500 nucleotides, and more typically, is at least about 5 nucleotides in length. The nucleic acid molecule can be derived from any source, including mammalian, fish, bacterial, insect, viral, plant, synthetic sources or combinations thereof. A nucleic acid molecule can be produced by methods commonly known in the art such as recombinant DNA technology (e.g., polymerase chain reaction (PCR), amplification, cloning) or chemical synthesis. Nucleic acid molecules include natural nucleic acid molecules and homologues thereof, including, but not limited to, natural allelic variants and modified nucleic acid molecules in which nucleotides have been inserted, deleted, substituted, or inverted in such a manner that such modifications do not substantially interfere with the nucleic acid molecule's ability to elicit an immune response useful in the methods of the present invention. A nucleic acid homologue may be produced using a number of methods known to those skilled in the art (see, for example, Sambrook et al., Molecular Cloning: A Laboratory Manual, Cold Spring Harbor Labs Press, 1989), which is incorporated herein by reference.

The terms "selectable marker" and "selectable marker gene" refer to a gene that encodes a product that protects the organism in which the gene is expressed from a selective agent (e.g., an antibiotic) or a condition that would normally kill the organism or inhibit its growth. Selectable marker genes are most commonly antibiotic resistance genes (e.g., kanamycin resistance genes, ampicillin resistance genes, chloramphenicol resistance genes, tetracycline resistance genes, etc.). Thus, for example, when *E. coli* cells are subjected to a transformation procedure to introduce a plasmid encoding a kanamycin resistance gene and then grown on or in media containing kanamycin, only the *E. coli* cells that have successfully taken up the plasmid and expressed the kanamycin resistance gene will survive. The terms "selectable marker" and "selectable marker gene" also include genes that code for enzymes involved in the synthesis of a compound that is essential for the growth of an organism. When introduced into an auxotrophic organism that is unable to synthesize the essential compound, such genes allow the organism to grow in a medium that has been supplemented with the essential compound. For example, bacterial cells that are auxotrophic for the amino acid lysine due to a mutation in or the absence of an enzyme involved in lysine biosynthesis normally are unable to grown on media that has not been supplemented with lysine. When such bacteria are subjected to a transformation procedure to introduce a plasmid encoding the enzyme involved in lysine biosynthesis, the bacteria that have successfully taken up the plasmid and expressed the enzyme will survive when grown on media that has not been supplemented with lysine. The terms "selectable marker" and "selectable marker gene" further include genes that allow for poison/antidote selection. For example, the *ccdB* gene encodes a protein that binds to DNA gyrase, an essential enzyme for cell division. Upon binding to DNA gyrase, the *ccdB* gene product impairs gene replication and induces cell death. Thus, bacterial expressing the *ccdB* gene product cannot survive. The *ccdA* gene encodes a protein (the "antidote") that acts as a natural inhibitor of the *ccdB* gene product. Thus, when bacteria having the *ccdB* gene in their bacterial genome are subjected to a transformation procedure to introduce a plasmid encoding the *ccdA* gene product, only the cells that successfully take up the plasmid and express the *ccdA* gene will survive.

The terms "screenable marker" and "screenable marker gene" refer to a gene that encodes a product that allows an observer to distinguish between cells expressing the screenable marker gene and cells that are not expressing the screenable marker gene. Screenable marker gene systems are well known in the art and include, for example, *lacZ* genes and genes encoding fluorescent proteins such as green fluorescent protein (GFP), yellow fluorescent protein (YFP), red fluorescent protein (RFP), blue fluorescent protein (BFP), or cyan fluorescent protein (CFP).

As used herein, the term "improving production" refers to an improvement in a measurement such as the health, weight, size, meat quality, or other parameter between fish receiving the immunomodulator composition and those fish not receiving the immunomodulator composition.

As used herein, the term "improving survivability" refers to improved survival of recipient fish compared to fish not receiving the immunomodulator composition.

As used herein, the term "fish" refers to any fish. Fish may be any member of such species, whether domestic or wild, and may be commercially reared for breeding, meat or egg production. Exemplary fish include, without limitation, any species of the Phylum Chordata, Sub Phylum Vertebrata, and Super Class Pisces. By further example, without limitation, such fish include catfish, channel catfish, black bullhead, yellow bullhead, brown bullhead, carp, crucian carp, trout, rainbow trout, brown trout, speckled brook trout, salmon, atlantic salmon, coho salmon, chinooK or king salmon, tench, roach, pike, pike-perch, dover sole, turbot, yellowtail, bass, smallmouth bass, largemouth bass, striped bass, stripers, milkfish, tilapia, gray mullet, eels, cod, and any other variety or aquatic species with which the present invention may be practiced will be apparent to those skilled in the art. In some aspects, fish may be diagnosed with an infectious disease, may be at risk for an infectious disease, or may be experiencing an infectious disease. Fish may be of any age including in ovo, new born, adolescence, adult, middle age, or elderly.

**Table 1: Plasmid DNA Sequences**

| **Plasmid** | **SEQ ID NO.** | **SEQUENCE** |
|---|---|---|
| pMB75.6 | 2 | |
| | | |
| pGCMB75.6 | 1 | |
| | | |
| | | |
| pLacZMB75.6 | 4 | |
| | | |

### EXAMPLES

The following non-limiting examples are provided to further illustrate the present invention.

### Example 1. Evaluation of Atlantic salmon receiving a DNA immunomodulator when formulated with inactivated IPNV antigen using an IPNV co-habitation challenge.

The purpose of this study was to determine the efficacy of the DNA immunomodulator administrated to salmon prior to IPNV co-habitation.

### Immunomodulators

The DNA immunomodulator used in this study was a plasmid DNA liposomal complex containing CpG dinucleotides sequences. The plasmid DNA is the pMB75.6 plasmid represented by SEQ ID NO: 2. The plasmid DNA liposomal complex was formulated in a 5% dextrose solution.

The Montanide oil used in this study (Montanide ISA 763 A) is a metabolizable oil that is a type I adjuvant providing a strong and long term immune response. Montanide ISA 763 A VG, supplied by Seppic, contains injectable, metabolizable oils (does not contains mineral oil) and an extremely refined emulsifier obtained from mannitol and purified oleic acid of vegetable origin.

### IPNV Antigen

The IPNV (Infectious pancreas necrosis virus) was propagated in the CHSE-214 cell line until 100% cytopathic effect (CPE) was attained. The antigen was formalin inactivated and then formulated in combination with either one of the immunomodulators listed above at a constant concentration across the various treatments.

### Alpha Ject® 2-2

This commercially available vaccine was used as a control. The vaccine is an oil emulsion used to prevent mortality in fish due to furunculose and IPNV infection.

### IPNV Antigen Vaccine Formulation

The IPNV antigen was formulated as a monovalent vaccine in combination with either the DNA immunomodulator or Montanide oil at three different concentrations/ratios. The treatment composition summary is shown in Table 2.

**Table 2. Vaccine Formulations.**

| **Treatment Group** | **Vaccine formulation** | | | | **Number of 0.05 ml doses** | **Final volume (ml)** |
|---|---|---|---|---|---|---|
| | **Antigen** | **Adjuvant** | | | | |
| | **IPNV (viral particles** | **DNA** | | **Montadine ISA 763 A VG** | | |
| | | **µg ml⁻¹** | **µg in 0.05 ml** | | | |
| A | 0 | 0 | 0 | 0 | 110 (90 + 20) | 5.5 |
| B | 0 | 2 | 0 | 0 | 110 (90 + 20) | 5.5 |
| c | 0 | 20 | 1 | 0 | 110 (90 + 20) | 5.5 |
| D | 0 | 200 | 1 | 0 | 110 (90 + 20) | 5.5 |
| E | 1.5x10 | 0 | 0 | 0 | 110 (90 + 20) | 5.5 |
| F | 1.5x10 | 2 | 0 | 0 | 120 (90 + 30) | 6 |
| G | 1.5x10 | 20 | 1 | 0 | 120 (90 + 30) | 6 |
| H | 1.5x10 | 200 | 1 | 0 | 120 (90 + 30) | 6 |
| I | 1.5x10 | 0 | 0 | 40:60 | 110 (90 + 20) | 5.5 |
| J | 1.5x10 | 0 | 0 | 50:50 | 110 (90 + 20) | 5.5 |
| K | 1.5x10 | 0 | 0 | 70:30 | 110 (90 + 20) | 5.5 |
| L | Alpha Ject® 2- | | | | 110 (90 + 20) | 11 |

### Study Animals

Atlantic salmon (*Salmo salar*) were obtained unvaccinated and immune status documented from the supplier. Fish sexually matured, injured or deformed were excluded from the study. The fish were divided into 12 treatment groups (treatment groups A through L) of 30 fish each in three tanks for a total of 360 treatment fish per tank. Another group consisting of 72 fish was used as shedders, or fish actively shedding IPNV, in each of the three tanks. Prior to administration of the vaccine, trial fish were anaesthetized and marked by injection of a PIT-tag two weeks before treatment day.

### Study Design

The fish were starved for a minimum of 48 hours prior to administration of the vaccine. The treatment groups were administered varying doses of the vaccine or control substances described above in Table 2 on the day of treatment. The vaccine or control substances were administered intraperitoneally according to Table 2. The study was performed in three tanks. Each tank had 12 experimental groups of 30 fish each. Two tanks were used as duplicates for the challenge experiment (Tanks 1 and 2, Table 3). The third tank was used for sampling during the immunomodulation study (Tank 3, Table 3).

**Table 3. Study Design. DNA immunomodulator is abbreviated DNA in this table.**

| **Group** | **Vaccine/control substance** | **No. of fish** | **Tank and study** |
|---|---|---|---|
| A | Dextrose 5% | 30 | Tank 1 - IPNV challenge for vaccine testing |
| B | DNA (2 µg ml⁻¹) | 30 | |
| c | DNA (20 µg ml⁻¹) | 30 | |
| D | DNA (200 µg ml⁻¹) | 30 | |
| E | IPNV antigen | 30 | |
| F | IPNV antigen/DNA (2 µg ml⁻¹) | 30 | |
| G | IPNV antigen/DNA (20 µg ml⁻¹) | 30 | Shedders injected with IPNV with a concentration of approx. 10⁷ TCID₅₀ ml⁻¹ |
| H | IPNV antigen/DNA (200 µg ml⁻¹) | 30 | |
| I | IPNV antigen/Montanide oil (40%) | 30 | |
| J | IPNV antigen/Montanide oil (50%) | 30 | |
| K | IPNV antigen/Montanide oil (70%) | 30 | |
| L | Commercial vaccine as per label | 30 | |
| Shedders | | 72 | |
| A | Dextrose 5% | 30 | Tank 2 - IPNV challenge for vaccine testing |
| B | DNA (2 µg ml⁻¹) | 30 | |
| c | DNA (20 µg ml⁻¹) | 30 | |
| D | DNA (200 µg ml⁻¹) | 30 | |
| E | IPNV antigen | 30 | |
| F | IPNV antigen/DNA (2 µg ml⁻¹) | 30 | |
| G | IPNV antigen/DNA (20 µg ml⁻¹) | 30 | Shedders injected with IPNV with a concentration of approx. 10⁷ TCID₅₀ ml⁻¹ |
| H | IPNV antigen/DNA (200 µg ml⁻¹) | 30 | |
| I | IPNV antigen/Montanide oil (40%) | 30 | |
| J | IPNV antigen/Montanide oil (50%) | 30 | |
| K | IPNV antigen/Montanide oil (70%) | 30 | |
| L | Commercial vaccine as per label | 30 | |
| Shedders | | 72 | |
| A | Dextrose 5% | 30 | Tank 3 - Immunomodulation study, Sampling tank |
| B | DNA (2 µg ml⁻¹) | 30 | |
| c | DNA (20 µg ml⁻¹) | 30 | |
| D | DNA (200 µg ml⁻¹) | 30 | |
| E | IPNV antigen | 30 | |
| F | IPNV antigen/DNA (2 µg ml⁻¹) | 30 | |
| G | IPNV antigen/DNA (20 µg ml⁻¹) | 30 | Shedders injected with IPNV with a concentration of approx. 10⁷ TCID₅₀ ml⁻¹ |
| H | IPNV antigen/DNA (200 µg ml⁻¹) | 30 | |
| I | IPNV antigen/Montanide oil (40%) | 30 | |
| J | IPNV antigen/Montanide oil (50%) | 30 | |
| K | IPNV antigen/Montanide oil (70%) | 30 | |
| L | Commercial vaccine as per label | 30 | |
| Shedders | | 48 | |

The challenge experiments were performed in connection with transfer to sea water after a minimum of 600 degree days, approximately 6 weeks post vaccination and photoperiod manipulation. The fish were starved for a minimum of 25 hours prior to challenge. All tanks were challenged at the same time. The shedders were intraperitoneally injected with the cultivated virus and marked by cutting the adipose fin at time of challenge. Following injection and marking, the shedders were introduced. Fish originally from Tanks 1 and 2 were allocated to tanks M5 and M6, and fish originally from Tank 3 were transferred to tank M2.

### Sampling

For each time point, fish were culled and tissue, e.g. head-kidney, liver and spleen, samples obtained for immunoprofiling. Blood was also collected for serology and/or transcriptome analysis. At each time point, 5 fish per treatment were culled and sampled. Before vaccination, 10 fish from the general population were sampled. After vaccination, samples from tank 3 were collected at 24 and 48 hours post-vaccination. After challenge, samples from tanks 1 and 2 were taken for standard confirmatory loss (10% of mortality). Samples from tank 3 were collected at 3 and 7 days post-challenge. At the end of the challenge period, the surviving fish were culled and sampled.

A representative selection of dead fish from the IPNV challenge tanks (tanks 1 and 2) were kidney sampled and tested in an IPNV Ag ELISA. The selection was done in the start, middle, and the end of the mortality up to a total of 10% of the fish (30/tank).

A representative selection of the dead fish during challenge (∼10% of the total number of fish included in the trial) were examined bacteriologically.

### Results

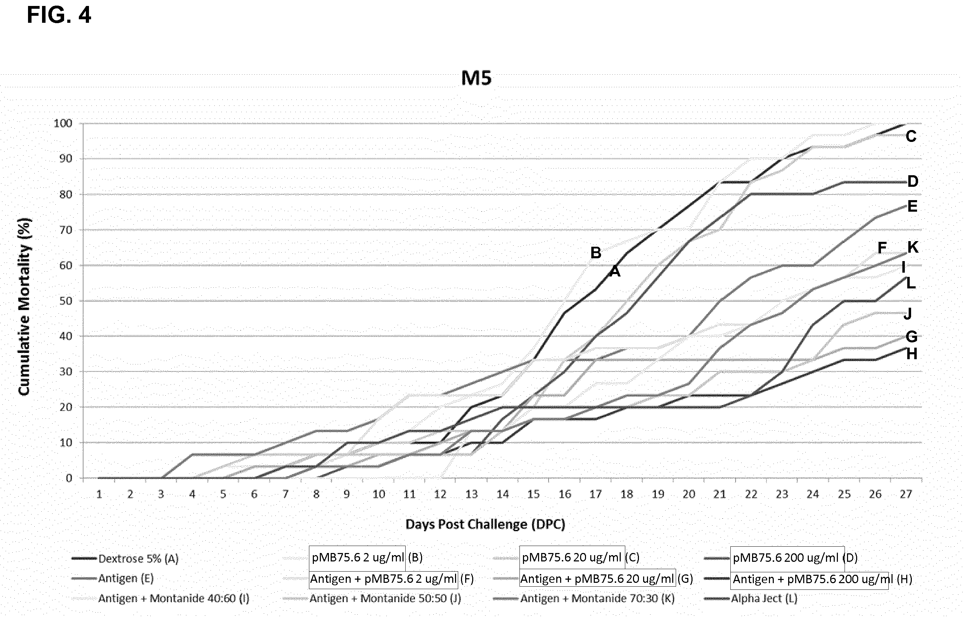

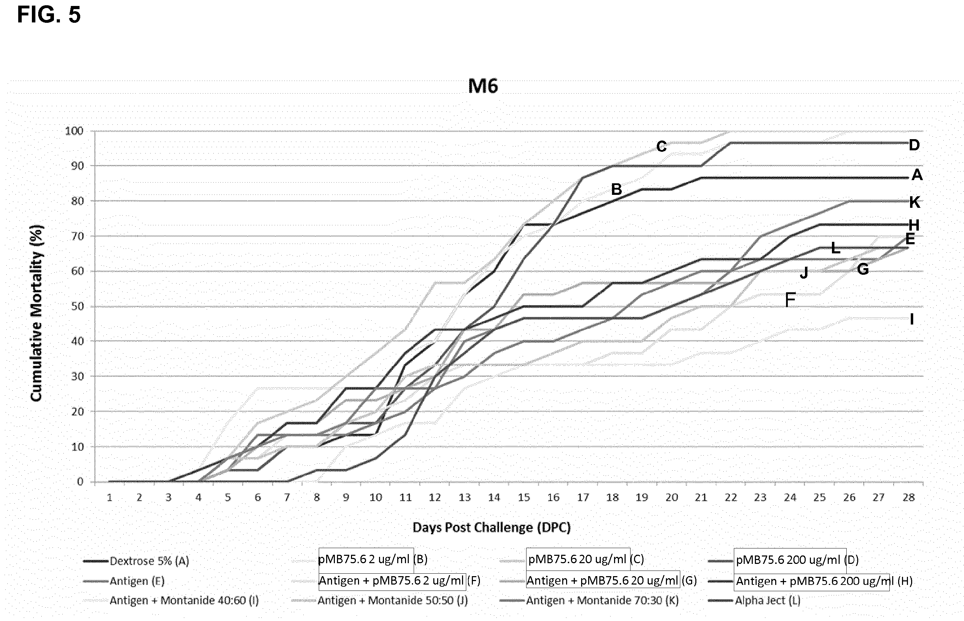

Following the introduction of the shedders, the challenge study was allowed to progress until mortality leveled off or reached 100% in the negative control groups. Plateaus in the mortality were reached between day 22 and 28 days post-infection in tanks 1 and 2 (FIG. 4 & FIG. 5).

Deceased fish were sampled using an IPNV Ag ELISA and bacteriological analysis. IPNV Ag ELISA testing revealed 75.5% of fish from tank M6 being positive for IPNV, 62.5% of fish in tank M5 and just 35.2% of fish in tank M2 (Table 4). Samples taken from 10% of the mortality were submitted for bacteriology analysis. The percentage of samples with no bacterial growth taken from fish in the various tanks were 77.5%, 64.9% and 98.1% for tanks M5, M6 and M2 respectively (Table 5). The majority of mortality recorded subsequently were negative for bacterial growth.

**Table 4. IPNV Ag ELISA results.**

| **Tank** | **Total No. fish** | **Total No.** |
|---|---|---|
| M5 | 40 (26.5%) | 25 (62.5%) |
| M6 | 57 (37.7%) | 43 (75.5%) |
| M2 | 54 (35.8%) | 19 (35.2%) |

**Table 5. Bacteriological results from tanks M5, M6 and M2.**

| **Tank** | **Date of sampling** | **Total No. fish tested** | **Total No. bacteriology samples negative** | **Total No. bacteriology samples positive** |
|---|---|---|---|---|
| M5 | 01/07/2014 | 3 | 3 | - |
| | 02/07/2014 | 5 | 4 | 1 |
| | 03/07/2014 | 3 | 3 | - |
| | 04/07/2014 | 1 | 1 | - |
| | 05/07/2014 | 4 | 4 | - |
| | 08/07/2014 | 7 | - | 7 |
| | 09/07/2014 | 5 | 5 | - |
| | 18/07/2014 | 11 | 11 | - |
| | 21/07/2014 | 1 | 0 | 1 |
| | **Total** | **40** | **31 (77.5%)** | **9 (22.5%)** |
| M6 | 01/07/2014 | 12 | 7 | 5 |
| | 08/07/2014 | 12 | - | 12 |
| | 13/07/2014 | 8 | 7 | 1 |
| | 14/07/2014 | 6 | 4 | 2 |
| | 15/07/2014 | 4 | 4 | |
| | 16/07/2014 | 8 | 8 | - |
| | 17/07/2014 | 5 | 5 | - |
| | 18/07/2014 | 2 | 2 | - |
| | **Total** | **57** | **37 (64.9%)** | **20 (35.1%)** |
| M2 | 03/07/2014 | 2 | 2 | - |
| | 04/07/2014 | 1 | 1 | - |
| | 05/07/2014 | 1 | 1 | - |
| | 10/07/2014 | 4 | 4 | - |
| | 15/07/2014 | 4 | 4 | - |
| | 23/07/2014 | 42 | 41 | 1 |
| | **Total** | **54** | **53 (98.1%)** | **1 (1.9%)** |

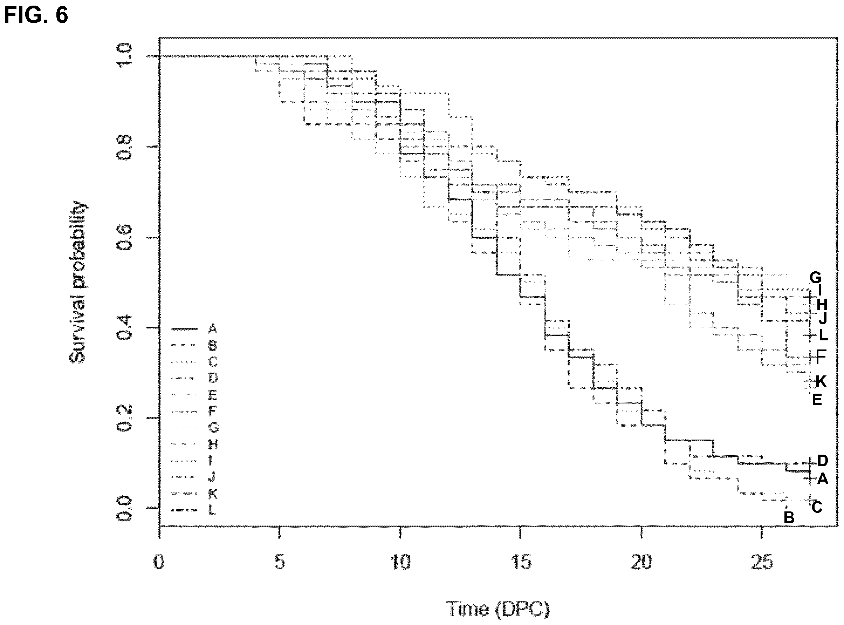

Kaplan-Meier survival curves of the combined data were determined in order to understand and compare the survival times from each treatment group (group A to L). As observed in FIG. 6, the survival probability was secluded in two distinct populations. The population with lower survival probability was composed of fish treated with dextrose (A) or the 3 different doses of the DNA immunomodulator without antigen (B, C and D) while the population showing a greater survival probability was composed of the groups treated with only IPNV antigen (E), the IPNV antigen formulated with the DNA immunomodulator (F, G and H) or with Montanide oil (I, J and K), and the commercial vaccine (L). When compared as a whole, statistically significant differences were found in the survival curves between the 12 different treatments (Chi- square = 110.6 df = 11; p-values (<0.0001) = 2.2x10-16).

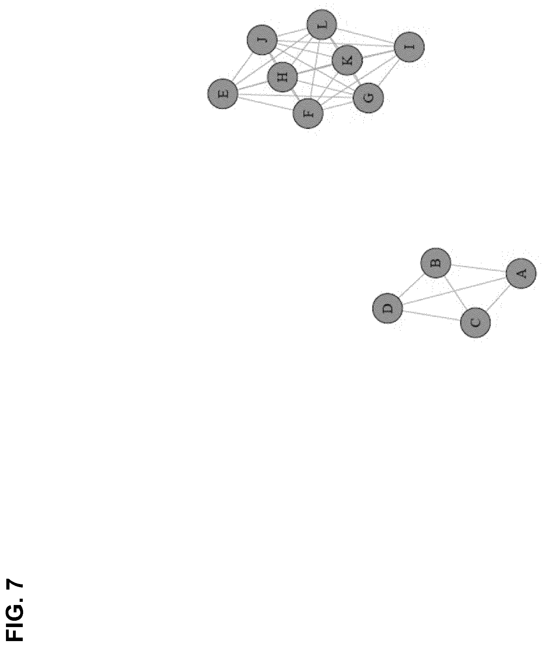

The treatments were divided among 2 distinct clusters (FIG. 7). One cluster included the dextrose- treatment (A) as well as all the DNA immunomodulator without antigen treatments (B, C, D). The other cluster was composed of fish treated with IPNV antigen only (E), the DNA immunomodulator formulated vaccines (F, G, H), the Montanide formulated antigen (I, J, K), and the commercial vaccine (L).

Significant difference between the two clusters was evident thus confirming that fish survival was significantly improved by vaccination compared to the negative control groups however no distinction was seen between the different vaccine formulations.

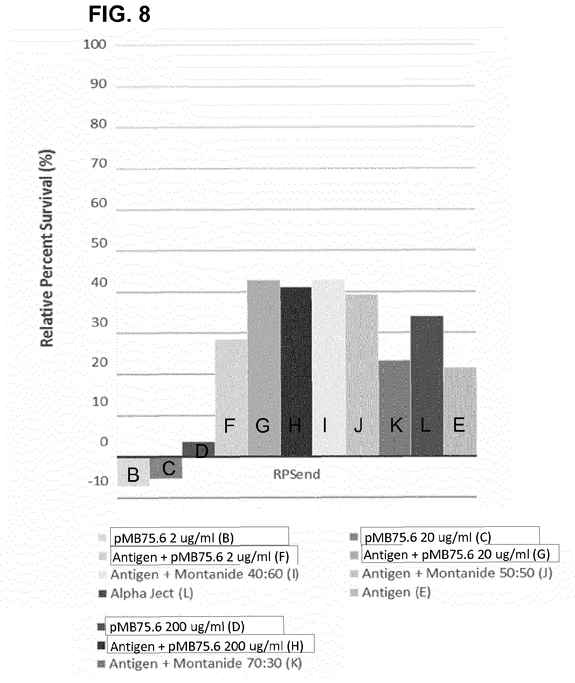

Relative percent survival was calculated in all treatment groups at the end (RPSend) of the experiment (FIG. 8). Significant differences were measured between 5% dextrose negative control group and all the formulated vaccines.

The Speilberg scale was used to access possible negative effects of the adjuvants used to formulate the vaccines. No particular secondary effects were observed. Tissue lesions were almost non-existent with only occasional level 2 (low) score for adhesions and pigmentation in the commercial product and level 1 scoring for the DNA immunomodulator (Table 6). No vaccine residues were detectable in any of the fish sampled.

**Table 6. Spielberg scoring for tanks M5 and M6.**

| **Tank** | **Gro** | **No. Fish** | **Visceral adhesions** | **Pigmentati on** | **Vaccine residues** |
|---|---|---|---|---|---|
| **M5** | E | 5 | 0 | 0 | 0 |
| | F | 7 | 0 | 0 | 0 |
| | G | 17 | 0 | 0 | 0 |
| | H | 16 | 1 in 2 | 1 in 1 fish | 0 |
| | I | 12 | 1 in 1 | 1 in 1 fish | 0 |
| | J | 14 | 0 | 0 | 0 |
| | K | 9 | 1 in 2 | 1 in 1 fish | 0 |
| | L | 9 | 1 in 4 fish | 1 in 7 fish | 0 |
| | | | | 2 in 2 fish | |
| **M6** | A | 1 | 0 | 3 in 1 fish | 0 |
| | E | 5 | 0 | 0 | 0 |
| | F | 7 | 0 | 0 | 0 |
| | G | 8 | 0 | 0 | 0 |
| | H | 5 | 1 in 1 | 0 | 0 |
| | I | 13 | 1 in 1 | 0 | 0 |
| | J | 8 | 0 | 1 in 1 fish | 0 |
| | K | 5 | 0 | 0 | 0 |
| | L | 10 | 1 in 7 fish | 1 in 5 fish | 0 |
| | | | | 2 in 1 fish | |
| **Combined data** | A | 1 | 0 | 3 in 1 fish | 0 |
| | B | 1 | 0 | 0 | 0 |
| | D | 1 | 0 | 0 | 0 |
| | E | 14 | 0 | 0 | 0 |
| | F | 21 | 0 | 0 | 0 |
| | G | 32 | 0 | 0 | 0 |
| | H | 19 | 1 in 3 | 1 in 1 fish | 0 |
| | I | 25 | 1 in 2 | 1 in 1 fish | 0 |
| | J | 22 | 0 | 1 in 1 fish | 0 |
| | K | 14 | 1 in 2 | 1 in 1 fish | 0 |
| | L | 19 | 1 in 11 fish | 1 in 12 fish | 0 |
| | | | 2 in 8 fish | 2 in 3 fish | |

### Conclusion

In this study, the efficacy of a DNA immunomodulator formulated with inactive IPNV as a vaccine in Atlantic salmon was assessed. When the DNA immunomodulator was administered alone (treatments B, C and D) it gave minimal protection, with mortality ranging between 93.3 to 100% and survival probabilities not significantly different than the dextrose-only control group. This was expected as the innate immune response elicited by the DNA immunomodulator would have most likely dissipated by the challenge time point (588 degree days or approximately 7 weeks post-vaccination) although this was not assessed in the current study.

As depicted in FIG. 8, the presence of the IPNV inactivated antigen alone was sufficient in significantly increasing the survival probability of vaccinated fish with a RPSend of 21.4% compared to the 5% dextrose group. The protection was further increased with the adjuvanted formulations of the DNA immunomodulator (F=28.7%, G=42.9%, H=41.1 %) and Montanide (1=42.9%, J=39.3%, K=23.2%). On the safety aspect, no issues were recorded and the DNA immunomodulator was deemed safe for use in fish.

This study showed the potential use of the DNA immunomodulator as a fish vaccine adjuvant, its ability to help induce an immune response in Atlantic salmon as well as its safety profile when administered at 10 ug or lower.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.
As various changes could be made in the above products, compositions, and methods without departing from the scope of the invention, it is intended that all matter contained in the above description and shown in the accompanying drawings shall be interpreted as illustrative of the invention, which is as defined in the claims.

## Claims

1. An immunomodulator composition for use with an immunogen in treating or preventing an infection of fish, wherein said immunomodulator composition comprises an immunostimulatory nucleic acid sequence having at least 80% sequence identity with a sequence selected from the group consisting of SEQ ID NO: 1, SEQ ID NO: 2, SEQ ID NO: 3, and SEQ ID NO: 4, and a liposome delivery vehicle comprising pairs of lipids selected from the group consisting of N-[1-(2,3-dioleyloxy)propyl]-N,N,N-trimethylammonium chloride (DOTMA) and cholesterol; N-[1-(2,3-dioleoyloxy)propyl]-N,N,N-trimethylammonium chloride (DOTAP) and cholesterol; 1-[2-(oleoyloxy)ethyl]-2-oleyl-3-(2-hydroxyethyl)imidazolinium chloride (DOTIM) and cholesterol; and dimethyldioctadecylammonium bromide (DDAB) and cholesterol.

2. An immunomodulator composition for use with an immunogen according to claim 1, wherein the fish is a salmon.

3. An immunomodulator composition for use with an immunogen according to claim 1, wherein the fish is a catfish.

4. An immunomodulator composition for use with an immunogen according to claim 1, wherein the fish is a carp.

5. An immunomodulator composition for use with an immunogen according to claim 1, wherein the fish is a trout.

6. An immunomodulator composition for use with an immunogen according to claim 1, wherein the fish is a yellow tail.

7. An immunomodulator composition for use with an immunogen according to claim 1, wherein the fish is a bass.

8. An immunomodulator composition for use with an immunogen according to any one of the previous claims, wherein the fish is an aqua culture raised fish.

9. An immunomodulator composition for use with an immunogen according to any one of the previous claims, wherein the immunomodulatory composition further comprises a pharmaceutically acceptable carrier.

10. An immunomodulator composition for use in improving an immune response of fish to a vaccine antigen, wherein the immunomodulator composition comprises an immunostimulatory nucleic acid sequence having at least 80% sequence identity with a sequence selected from the group consisting of SEQ ID NO: 1, SEQ ID NO: 2, SEQ ID NO: 3, and SEQ ID NO: 4, and a liposome delivery vehicle comprising pairs of lipids selected from the group consisting of N-[1-(2,3-dioleyloxy)propyl]-N,N,N-trimethylammonium chloride (DOTMA) and cholesterol; N-[1-(2,3-dioleoyloxy)propyl]-N,N,N-trimethylammonium chloride (DOTAP) and cholesterol; 1-[2-(oleoyloxy)ethyl]-2-oleyl-3-(2-hydroxyethyl)imidazolinium chloride (DOTIM) and cholesterol; and dimethyldioctadecylammonium bromide (DDAB) and cholesterol.

## Patentansprüche

1. Immunmodulatorzusammensetzung zur Verwendung mit einem Immunogen beim Behandeln oder Verhindern einer Infektion von Fischen, wobei die Immunmodulatorzusammensetzung eine immunstimulatorische Nukleinsäuresequenz mit mindestens 80% Sequenzidentität mit einer Sequenz, die aus der Gruppe bestehend aus SEQ ID Nr. 1, SEQ ID Nr. 2, SEQ ID Nr. 3 und SEQ ID Nr. 4 ausgewählt ist, und ein Liposomabgabevehikel umfasst, das Paare von Liposomen umfasst, die aus der Gruppe bestehend aus N-[1-(2,3-Dioleyloxy)propyl]-N,N,N-trimethylammoniumchlorid (DOTMA) und Cholesterin; N-[1-(2,3-Dioleyloxy)propyl]-N,N,N-trimethylammoniumchlorid (DOTAP) und Cholesterin; 1-[2-(Oleoyloxy)ethyl]-2-oleyl-3-(2-hydroxyethyl)imidazoliniumchlorid (DOTIM) und Cholesterin und Dimethyldioctadecylammoniumbromid (DDAB) und Cholesterin ausgewählt sind.

2. Immunmodulatorzusammensetzung zur Verwendung mit einem Immunogen nach Anspruch 1, wobei der Fisch ein Lachs ist.

3. Immunmodulatorzusammensetzung zur Verwendung mit einem Immunogen nach Anspruch 1, wobei der Fisch ein Wels ist.

4. Immunmodulatorzusammensetzung zur Verwendung mit einem Immunogen nach Anspruch 1, wobei der Fisch ein Karpfen ist.

5. Immunmodulatorzusammensetzung zur Verwendung mit einem Immunogen nach Anspruch 1, wobei der Fisch eine Forelle ist.

6. Immunmodulatorzusammensetzung zur Verwendung mit einem Immunogen nach Anspruch 1, wobei der Fisch eine Gelbschwanzmakrele ist.

7. Immunmodulatorzusammensetzung zur Verwendung mit einem Immunogen nach Anspruch 1, wobei der Fisch ein Barsch ist.

8. Immunmodulatorzusammensetzung zur Verwendung mit einem Immunogen nach einem der vorhergehenden Ansprüche, wobei der Fisch ein in einer Aquakultur gezüchteter Fisch ist.

9. Immunmodulatorzusammensetzung zur Verwendung mit einem Immunogen nach einem der vorhergehenden Ansprüche, wobei die immunmodulatorische Zusammensetzung weiterhin einen pharmazeutisch annehmbaren Trägerstoff umfasst.

10. Immunmodulatorzusammensetzung zur Verwendung beim Verbessern einer Immunantwort von Fischen auf ein Impfstoff-Antigen, wobei die Immunmodulatorzusammensetzung eine immunstimulatorische Nukleinsäuresequenz mit mindestens 80% Sequenzidentität mit einer Sequenz, die aus der Gruppe bestehend aus SEQ ID Nr. 1, SEQ ID Nr. 2, SEQ ID Nr. 3 und SEQ ID Nr. 4 ausgewählt ist, und ein Liposomabgabevehikel umfasst, das Paare von Liposomen umfasst, die aus der Gruppe bestehend aus N-[1-(2,3-Dioleyloxy)propyl]-N,N,N-trimethylammoniumchlorid (DOTMA) und Cholesterin; N-[1-(2,3-Dioleyloxy)propyl]-N,N,N-trimethylammoniumchlorid (DOTAP) und Cholesterin; 1-[2-(Oleoyloxy)ethyl]-2-oleyl-3-(2-hydroxyethyl)imidazoliniumchlorid (DOTIM) und Cholesterin und Dimethyldioctadecylammoniumbromid (DDAB) und Cholesterin ausgewählt sind.

## Revendications

1. Composition d'immunomodulateur destinée à être utilisée avec un immunogène dans le traitement ou la prévention d'une infection chez un poisson, dans laquelle ladite composition d'immunomodulateur comprend une séquence d'acide nucléique immunostimulante ayant une identité de séquence d'acide d'au moins 80 % avec une séquence choisie dans le groupe constitué par la SEQ ID NO : 1, la SEQ ID NO : 2, la SEQ ID NO : 3 et la SEQ ID NO : 4, et un véhicule de délivrance de type liposome comprenant des paires de lipides choisies dans le groupe constitué par le chlorure de N-[1-(2,3-dioléyloxy)propyl]-N,N,N-triméthylammonium (DOTMA) et le cholestérol ; le chlorure de N-[1-(2,3-dioléyloxy)propyl]-N,N,N-triméthylammonium (DOTAP) et le cholestérol ; le chlorure de 1-[2-(oléoyloxy)éthyl]-2-oléyl-3-(2-hydroxyéthyl)imidazolinium (DOTIM) et le cholestérol ; et le bromure de diméthyldioctadécylammonium (DDAB) et le cholestérol.

2. Composition d'immunomodulateur destinée à être utilisée avec un immunogène selon la revendication 1, dans laquelle le poisson est un saumon.

3. Composition d'immunomodulateur destinée à être utilisée avec un immunogène selon la revendication 1, dans laquelle le poisson est un poisson-chat.

4. Composition d'immunomodulateur destinée à être utilisée avec un immunogène selon la revendication 1, dans laquelle le poisson est une carpe.

5. Composition d'immunomodulateur destinée à être utilisée avec un immunogène selon la revendication 1, dans laquelle le poisson est une truite.

6. Composition d'immunomodulateur destinée à être utilisée avec un immunogène selon la revendication 1, dans laquelle le poisson est une sériole.

7. Composition d'immunomodulateur destinée à être utilisée avec un immunogène selon la revendication 1, dans laquelle le poisson est un achigan.

8. Composition d'immunomodulateur destinée à être utilisée avec un immunogène selon l'une quelconque des revendications précédentes, dans laquelle le poisson est un poisson d'aquaculture.

9. Composition d'immunomodulateur destinée à être utilisée avec un immunogène selon l'une quelconque des revendications précédentes, dans laquelle la composition immunomodulatrice comprend en outre un véhicule pharmaceutiquement acceptable.

10. Composition d'immunomodulateur destinée à être utilisée dans l'amélioration de la réponse immunitaire d'un poisson à un antigène de vaccin, dans laquelle la composition d'immunomodulateur comprend une séquence d'acide nucléique immunostimulante ayant une identité de séquence d'acide d'au moins 80 % avec une séquence choisie dans le groupe constitué par la SEQ ID NO : 1, la SEQ ID NO : 2, la SEQ ID NO : 3 et la SEQ ID NO : 4, et un véhicule de délivrance de type liposome comprenant des paires de lipides choisies dans le groupe constitué par le chlorure de N-[1-(2,3-dioléyloxy)propyl]-N,N,N-triméthylammonium (DOTMA) et le cholestérol ; le chlorure de N-[1-(2,3-dioléyloxy)propyl]-N,N,N-triméthylammonium (DOTAP) et le cholestérol ; le chlorure de 1-[2-(oléoyloxy)éthyl]-2-oléyl-3-(2-hydroxyéthyl)imidazolinium (DOTIM) et le cholestérol ; et le bromure de diméthyldioctadécylammonium (DDAB) et le cholestérol.
